(19) **Europäisches Patentamt / European Patent Office / Office européen des brevets**

(11) **EP 4 678 649 A1**

(12) **EUROPEAN PATENT APPLICATION**

(43) Date of publication:
**14.01.2026 Bulletin 2026/03**

(21) Application number: **24306164.5**

(22) Date of filing: **10.07.2024**

(51) International Patent Classification (IPC):
**C07K 1/14** $^{(2006.01)}$

(52) Cooperative Patent Classification (CPC):
**C07K 1/14; A61L 2/0088; A61L 2/18; C12N 7/00;**
A61L 2/0023; A61L 2/0035; A61L 2/0047;
A61L 2/04; A61L 2/081; A61L 2/10; A61L 2202/21;
A61L 2202/22; C12N 2740/13061;
C12N 2740/13063

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **ROQUETTE FRERES**
**62136 Lestrem (FR)**

(72) Inventors:
• **PENG, Tao**
**138667 SINGAPORE (SG)**
• **HONG, Shiqi**
**138667 SINGAPORE (SG)**

(74) Representative: **Plasseraud IP**
**104 Rue de Richelieu**
**CS92104**
**75080 Paris Cedex 02 (FR)**

(54) **USE OF SUBSTITUTED CYCLODEXTRIN FOR VIRAL INACTIVATION IN BIOMANUFACTURING**

(57)    The invention relates to a method for inactivating an enveloped virus in a biopharmaceutical-containing fluid, said method comprising exposing said fluid to a substituted cyclodextrin. The invention also relates to a method for purifying and/or producing a biopharmaceutical comprising exposing a biopharmaceutical-containing fluid to a substituted cyclodextrin.

EP 4 678 649 A1

## Description

### Technical Field

The invention relates to compounds for use in viral inactivation, in methods for purifying or producing biopharmaceuticals.

### Background Art

Manufacturing therapeutic biological drugs processes carries an inherent risk of transmitting viral contaminants. Such contaminants can come from many sources, including starting materials, the use of reagents of animal origin during manufacture, and through contamination of the manufacturing system due to failures in the GMP process. As such, regulatory authorities recommend that biomanufacturing processes have dedicated viral inactivation and virus removal steps and request manufacturers validate the removal and inactivation of viruses from all biological products.

Various methods can be employed for viral inactivation and include heat inactivation/pasteurization, pH inactivation, UV and gamma ray irradiation, use of high intensity broad spectrum white light, addition of chemical inactivating agents, surfactants, and solvent/detergent treatments. Surfactants, such as detergents, solubilize membranes and therefore can be very effective in specifically inactivating enveloped viruses.

Solvent/detergent method is used for low-pH sensitive biologics to inactivate the enveloped virus. Detergents solubilize outer membranes of enveloped viruses by disrupting the protein layer with detergent micelles. Historically, non-ionic detergents, such as the commonly used Triton X-100, have been used to inactivate virus because they do not affect the therapeutic proteins of interest. However, ecological concerns over use of certain detergents, such as Triton X-100, have driven the search to identify more "eco-friendly" detergents that can be used to inactivate enveloped virus in biomanufacturing processes.

A few solutions have been proposed to replace Triton X-100, but which are suffering from the following deficiencies:

- iso-octyl cyclohexyl ether lacks efficiency,
- Polysorbate 80 has an efficiency which varies,
- Nereid is a new chemical that lacks safety data,
- PEG 9 Lauryl Ether is a semi-solid cream which makes it difficult to handle,
- PEG 6 Caprylic is turbid at low concentration and parenteral use thereof is not documented.

Therefore, it remains an unsatisfied need for a Triton X-100 substitute that is efficient enough, safe, and easy to handle.

### Technical Problem

It was thus an object of the present invention to provide an alternative to Triton X-100 for viral inactivation in biopharmaceutical manufacturing.

It was another object of the present invention to provide a viral inactivation solution that is safe.

It was another object of the present invention to provide a viral inactivation solution that is easy to handle.

### Presentation of the Invention

The inventors now have found that substituted cyclodextrin (e.g., hydroxypropyl-beta-cyclodextrin (HPBCD)) could successfully be used as a Triton X-100 substitute for inactivating enveloped viruses in biomanufacturing (Example section A and B).

Advantageously, the substituted cyclodextrin can be added at early stage of the biomanufacturing process (for example in the clarified cell harvest fluid) without disturbing the foregoing process steps (Example section C). As substituted cyclodextrins can be safely administered parenterally, it may even be safely left into the final drug formulation.

A further advantage is that substituted cyclodextrins may act as biopharmaceutical stabilizer, in particular where the biopharmaceutical is a therapeutic protein, thus also protecting the latter from stresses induced by the various manufacturing process steps.

### Brief Description of the Invention

The invention first relates to a method for inactivating an enveloped virus in a biopharmaceutical-containing fluid, said method comprising exposing said fluid to a substituted cyclodextrin.

Preferably, said exposure occurs at a substituted cyclodextrin concentration equal to or higher than 1% (w/v). Preferably, said biopharmaceutical-containing fluid is exposed to said substituted cyclodextrin for at least 1 minute. Preferably, said exposure of said biopharmaceutical-containing fluid to the substituted cyclodextrin occurs at a temperature higher than 5°C and lower than 50°C. Preferably, said exposure of said biopharmaceutical-containing fluid to the substituted cyclodextrin occurs at pH selected from 5 to 9. Preferably, said substituted cyclodextrin is substituted with alkyl groups, hydroxyalkyl groups, or sulfoalkyl groups. Preferably, said substituted cyclodextrin is substituted with alkyl groups, hydroxyalkyl groups, or sulfoalkyl groups having 1 to 5 carbon atoms. Preferably, said substituted cyclodextrin is selected from hydroxypropyl-cyclodextrin, methyl-cyclodextrin, sulfobutyl ether cyclodextrin, or from a mixture thereof. Preferably, said substituted cyclodextrin is selected from hydroxypropyl-beta-cyclodextrin (HPBCD), methyl-beta-cyclodextrin, sulfobutyl ether beta-cyclodextrin, or from a mixture thereof. Preferably, said substituted cyclodextrin comprises HPBCD. Preferably, said biopharmaceutical is a therapeutic protein. Preferably said method excludes the use of Triton X-100.

The invention also relates to a method for purifying a biopharmaceutical comprising:

1. subjecting a biopharmaceutical-containing fluid through unit operations, said unit operations including:

- harvest and clarification,
- viral inactivation,
- capture,
- polish chromatography,
- filtering;

wherein at least one of a unit operation including viral inactivation comprises exposing the fluid to a substituted cyclodextrin;
and wherein harvest and clarification occur before a unit operation comprising capture;
and wherein capture occurs before a unit operation comprising polish chromatography and before a unit operation comprising filtering;

2. recovering a purified biopharmaceutical.

Preferably, said substituted cyclodextrin is substituted with alkyl groups, hydroxyalkyl groups, or sulfoalkyl groups. Preferably, said substituted cyclodextrin is substituted with alkyl groups, hydroxyalkyl groups, or sulfoalkyl groups having 1 to 5 carbon atoms. Preferably, said substituted cyclodextrin is selected from hydroxypropyl-cyclodextrin, methyl-cyclodextrin, sulfobutyl ether cyclodextrin, or from a mixture thereof. Preferably, said substituted cyclodextrin is selected from hydroxypropyl-beta-cyclodextrin (HPBCD), methyl-beta-cyclodextrin, sulfobutyl ether beta-cyclodextrin, or from a mixture thereof. Preferably, said substituted cyclodextrin comprises HPBCD. Preferably, said biopharmaceutical is a therapeutic protein. Preferably said method excludes the use of Triton X-100.

The invention also relates to a method for producing a purified biopharmaceutical comprising:

1. producing a biopharmaceutical-containing fluid;
2. processing said fluid through unit operations, said unit operations including:

- harvest and clarification,
- viral inactivation,
- capture,
- polish chromatography,
- filtering;

wherein at least one of a unit operation including viral inactivation comprises exposing the fluid a substituted cyclodextrin;
and wherein harvest and clarification occur before a unit operation comprising capture;
and wherein capture occurs before a unit operation comprising polish chromatography and before a unit operation comprising filtering;

3. recovering a purified biopharmaceutical.

Preferably, said substituted cyclodextrin is substituted with alkyl groups, hydroxyalkyl groups, or sulfoalkyl groups. Preferably, said substituted cyclodextrin is substituted with alkyl groups, hydroxyalkyl groups, or sulfoalkyl groups having 1 to 5 carbon atoms. Preferably, said substituted cyclodextrin is selected from hydroxypropyl-cyclodextrin, methyl-cyclodextrin, sulfobutyl ether cyclodextrin, or from a mixture thereof. Preferably, said substituted cyclodextrin is selected from hydroxypropyl-beta-cyclodextrin (HPBCD), methyl-beta-cyclodextrin, sulfobutyl ether beta-cyclodextrin, or from a mixture thereof. Preferably, said substituted cyclodextrin comprises HPBCD. Preferably, said biopharmaceutical is a therapeutic protein. Preferably said method excludes the use of Triton X-100.

**Brief Description of Drawings**

Other features, details and advantages will be shown in the following detailed description and on the figures, on which:

**Fig. 1**

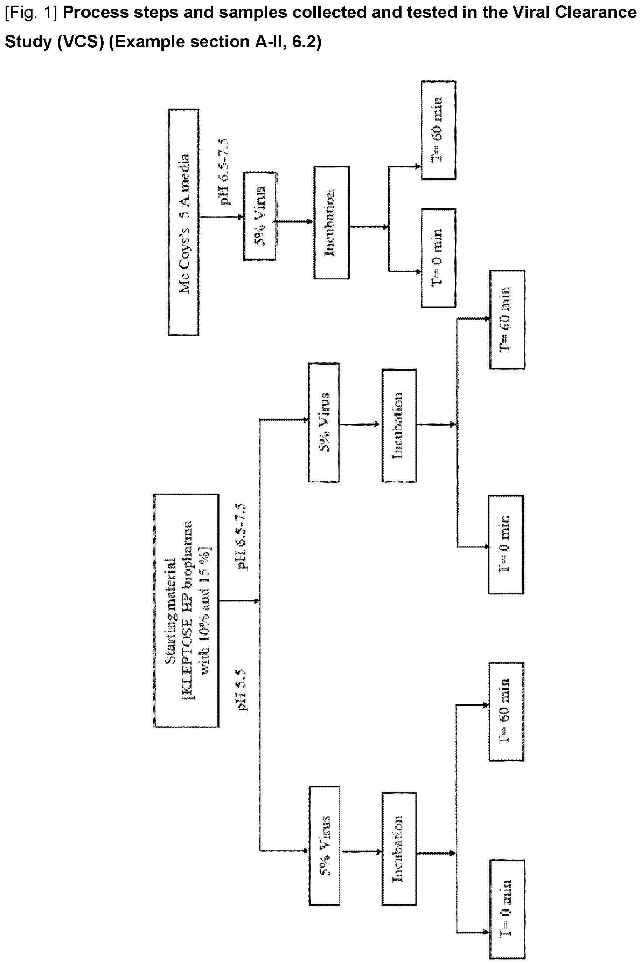

[Fig. 1] is a scheme of the process steps and samples collected and tested in the Viral Clearance Study (VCS) (Example section A-II, 6.2).
**Fig. 2**

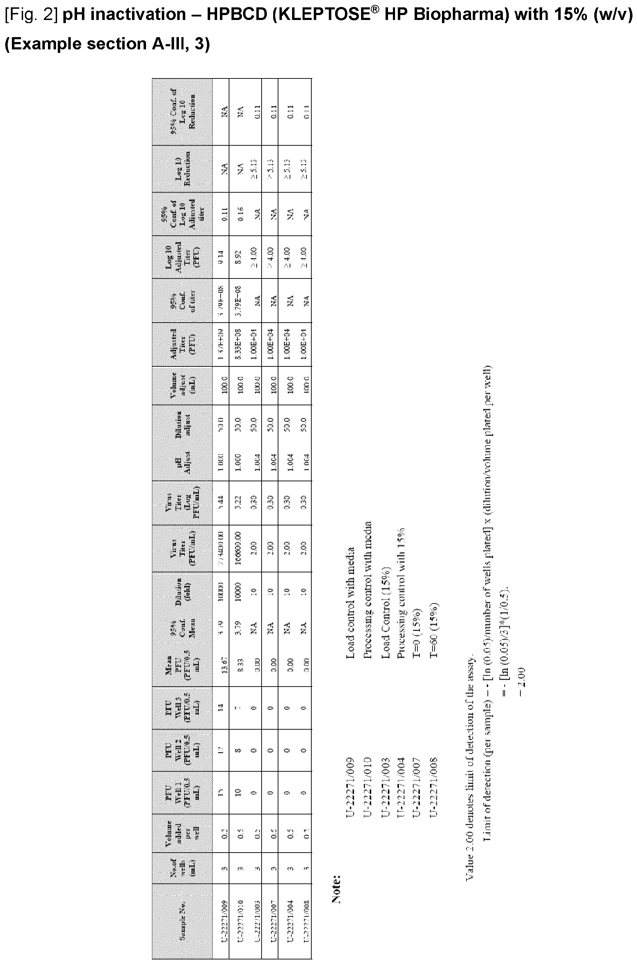

[Fig. 2] is a table showing the results of the VCS when using 15% (w/v) HPBCD (Example section A-III, 3).
**Fig. 3**

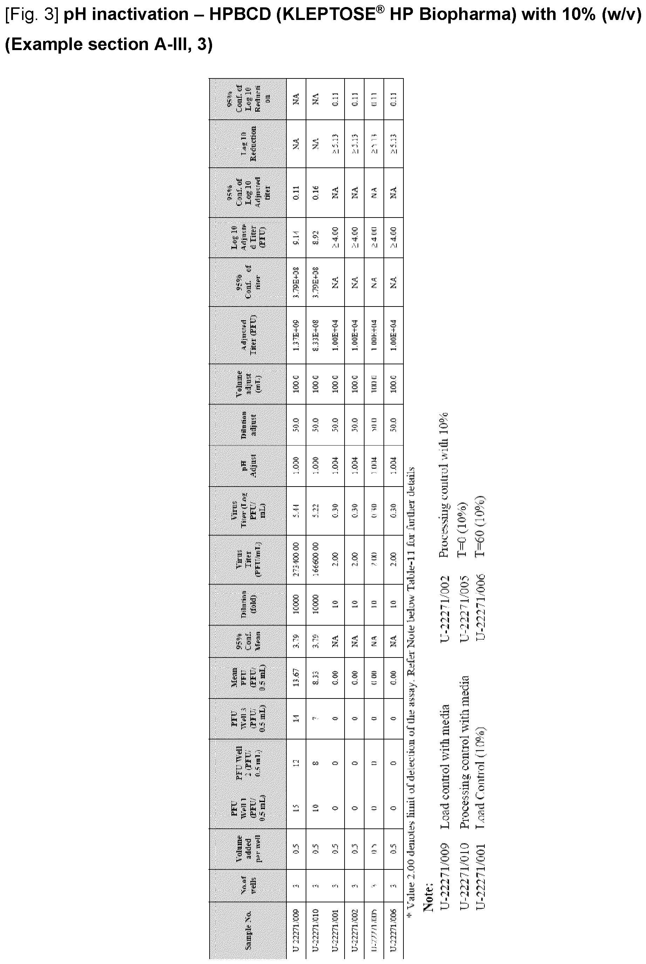

[Fig. 3] is a table showing the results of the VCS when using 10% (w/v) HPBCD (Example section A-III, 3).
**Fig. 4**

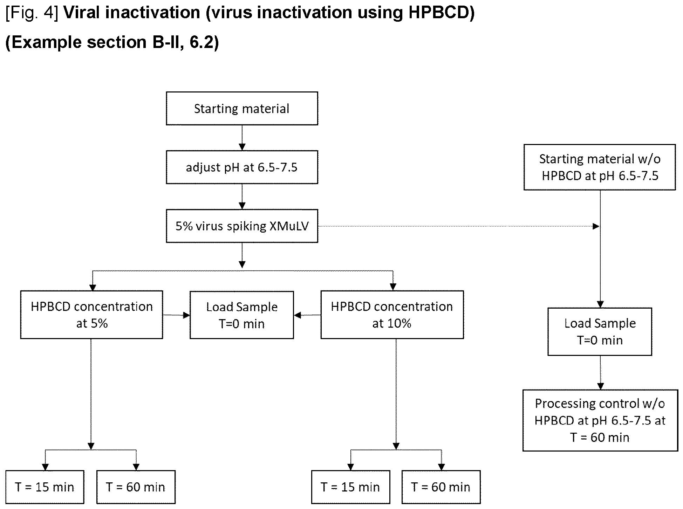

[Fig. 4] is a scheme of the process steps and samples collected and tested in the Viral Clearance Study (VCS) (Example section B-II, 6.2).
**Fig. 5**

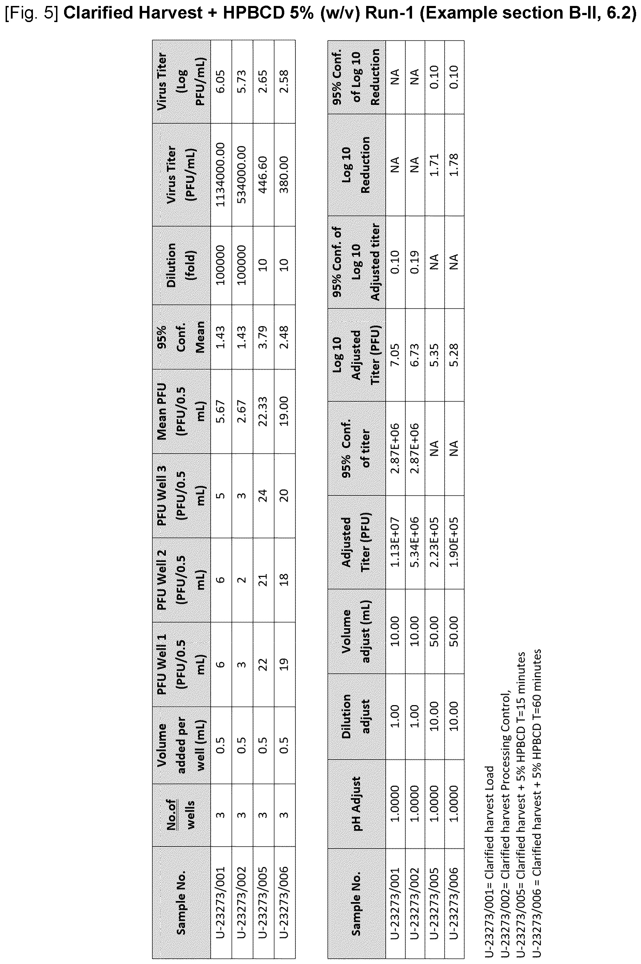

[Fig. 5] is a table showing the Run-1 results of the VCS when using 5% (w/v) HPBCD in clarified harvest fluid (Example section B-III, 6.2).
**Fig. 6**

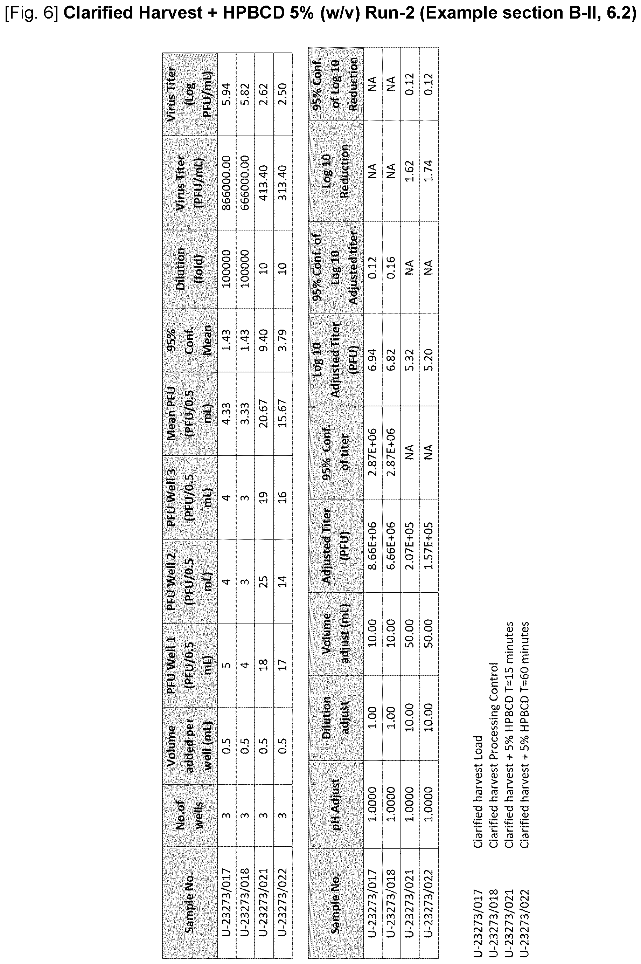

[Fig. 6] is a table showing the Run-2 results of the VCS when using 5% (w/v) HPBCD in clarified harvest fluid (Example section B-III, 6.2).
**Fig. 7**

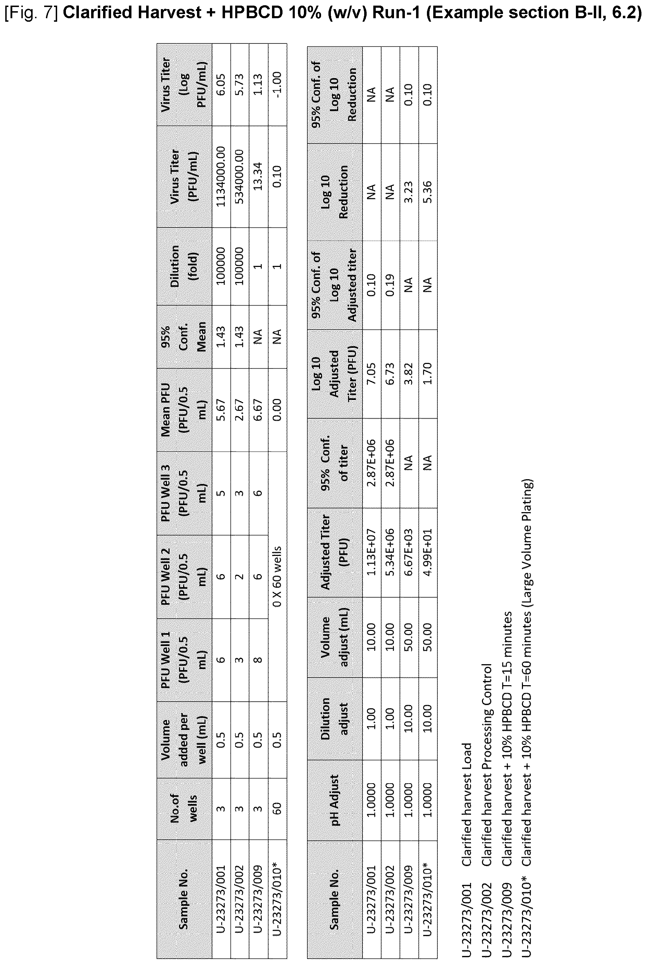

[Fig. 7] is a table showing the Run-1 results of the VCS when using 10% (w/v) HPBCD in clarified harvest fluid (Example section B-III, 6.2).
**Fig. 8**

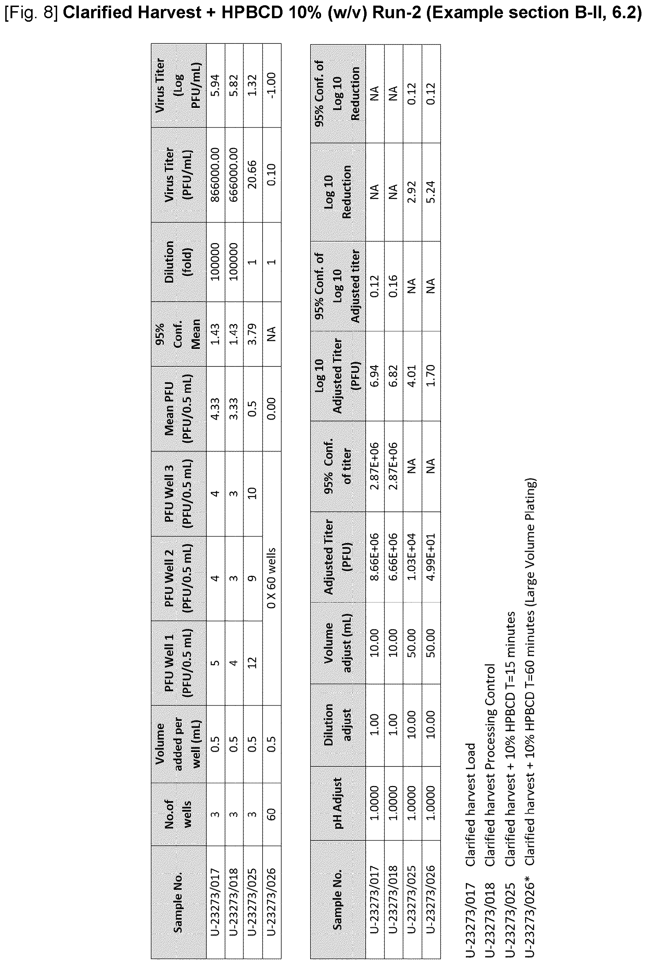

[Fig. 8] is a table showing the Run-2 results of the VCS when using 10% (w/v) HPBCD in clarified harvest fluid (Example section B-III, 6.2).
**Fig. 9**

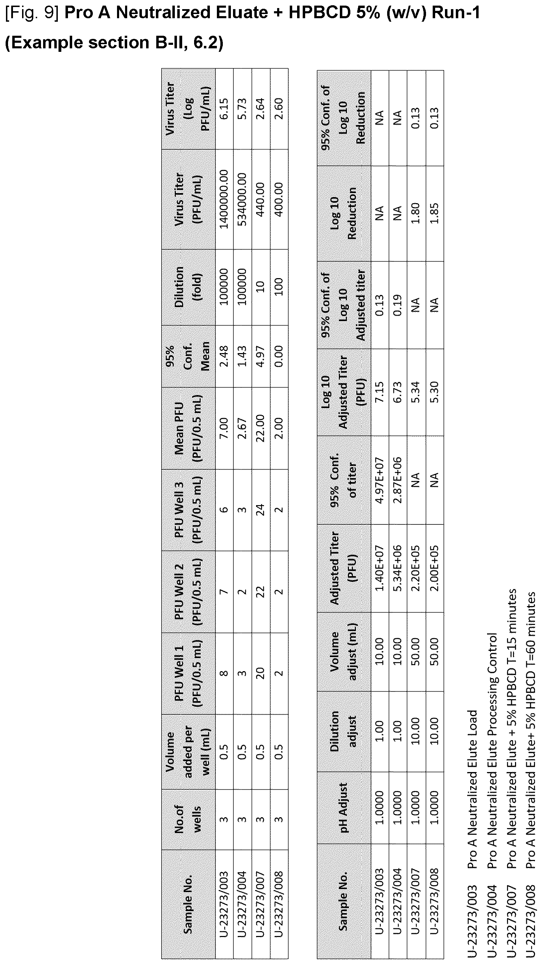

[Fig. 9] is a table showing the Run-1 results of the VCS when using 5% (w/v) HPBCD in Protein A affinity chromatography eluate (Example section B-III, 6.2).
**Fig. 10**

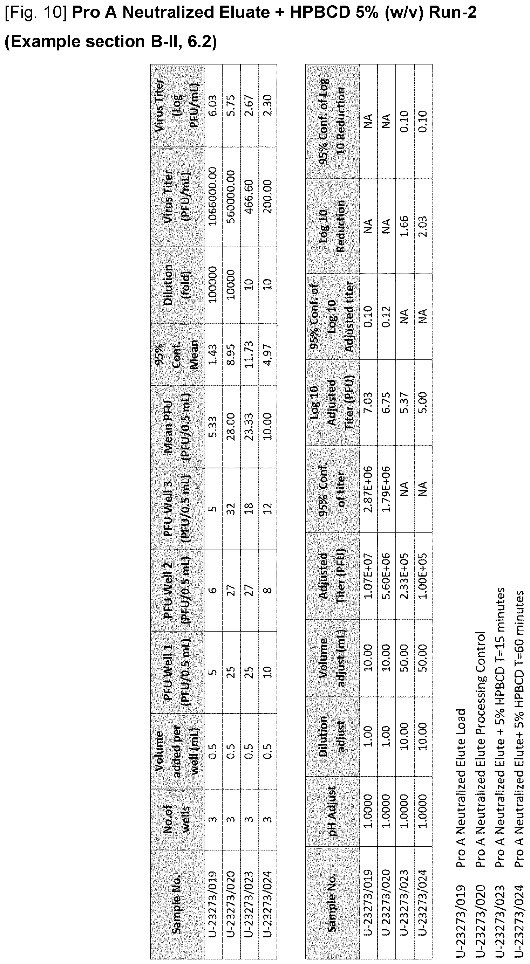

[Fig. 10] is a table showing the Run-2 results of the VCS when using 5% (w/v) HPBCD in Protein A affinity chromatography eluate (Example section B-III, 6.2).
**Fig. 11**

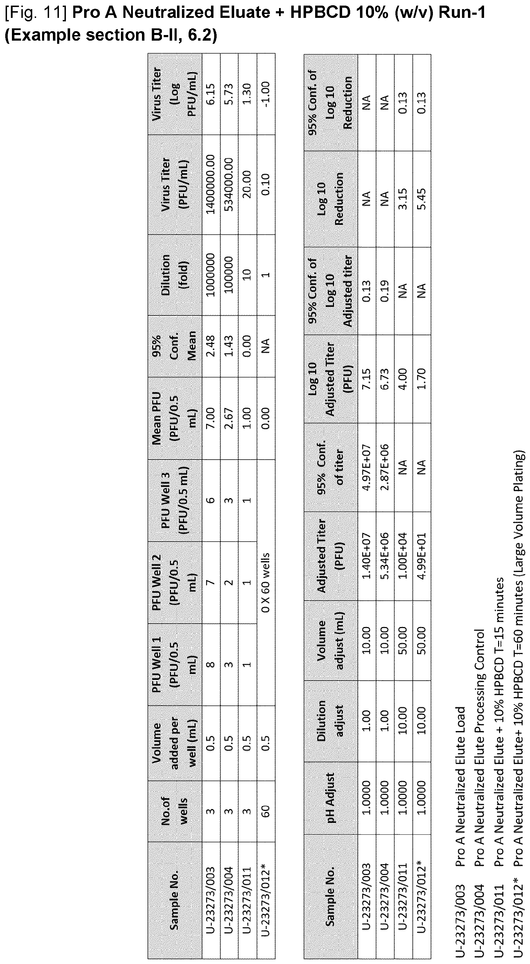

[Fig. 11] is a table showing the Run-1 results of the VCS when using 10% (w/v) HPBCD in Protein A affinity chromatography eluate (Example section B-III, 6.2).
**Fig. 12**

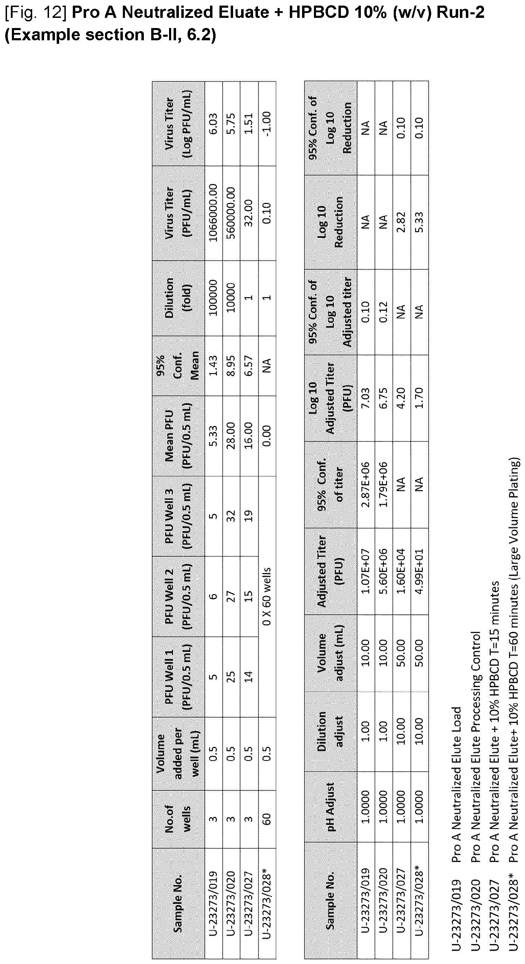

[Fig. 12] is a table showing the Run-2 results of the VCS when using 10% (w/v) HPBCD in Protein A affinity chromatography eluate (Example section B-III, 6.2).
**Fig. 13**

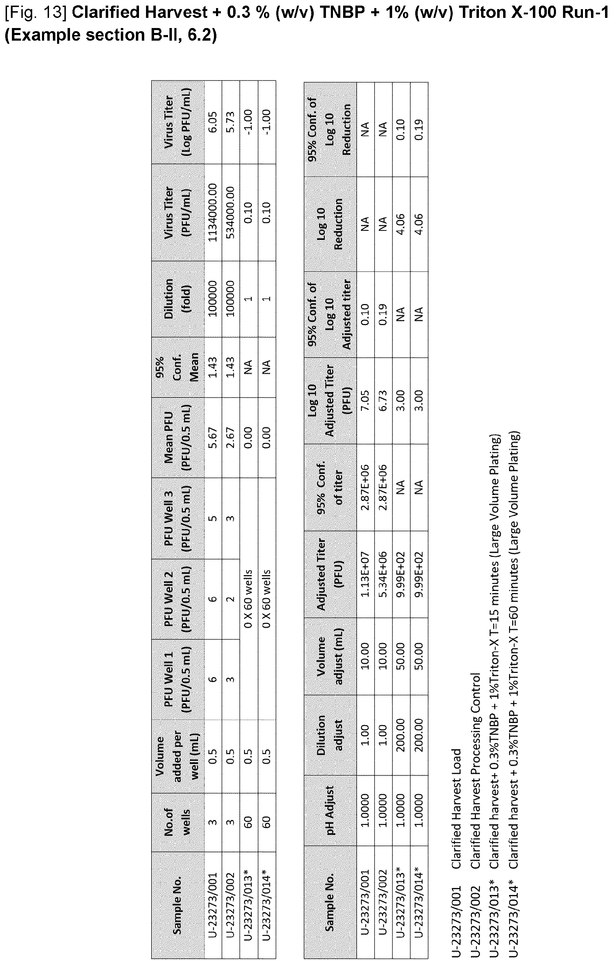

[Fig. 13] is a table showing the Run-1 results of the VCS when using 0.3 % (w/v) TNBP + 1% (w/v) Triton X-100 in clarified harvest fluid (Example section B-III, 6.2).
**Fig. 14**

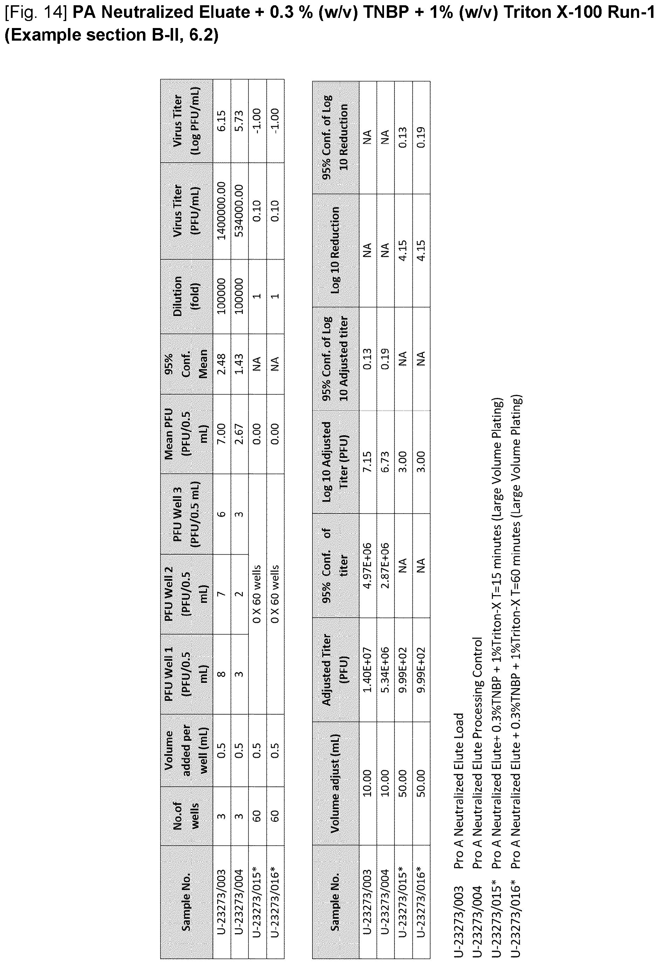

[Fig. 14] is a table showing the Run-1 results of the VCS when using 0.3 % (w/v) TNBP + 1% (w/v) Triton X-100 in Protein A affinity chromatography eluate (Example section B-III, 6.2).
**Fig. 15**

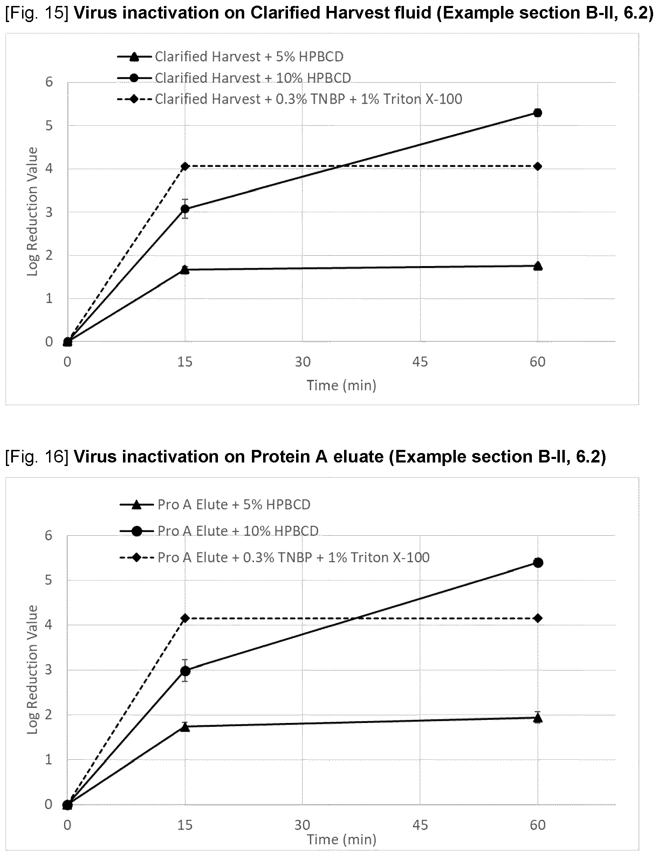

[Fig. 15] is a graph showing the Log10 reduction values (LRV) obtained when clarified harvest fluid is exposed to 5% or 10% (w/v) HPBCD for 15 or 60 minutes (Example section B-III, 6.2).
**Fig. 16**
[Fig. 16] is a graph showing the LRV obtained when Protein A affinity chromatography eluate is exposed to 5 or 10% (w/v) HPBCD for 15 or 60 minutes (Example section B-III, 6.2).
**Fig. 17**

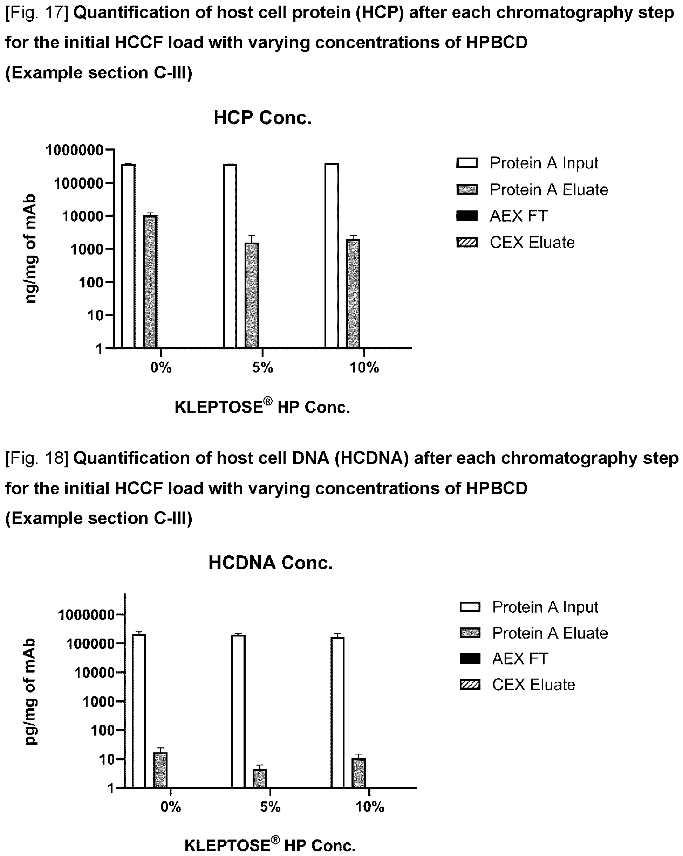

[Fig. 17] is a bar chart showing the quantification of host cell protein (HCP) after each chromatography step for the initial HCCF load with varying concentrations of HPBCD (Example section C-III).

**Fig. 18**

[Fig. 18] is a bar chart showing the quantification of host cell DNA (HCDNA) after each chromatography step for the initial HCCF load with varying concentrations of HPBCD (Example section C-III).

**Fig. 19**

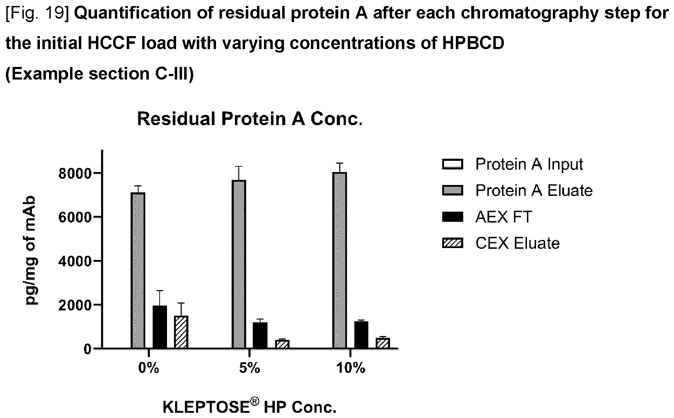

[Fig. 19] is a bar chart showing the quantification of residual protein A after each chromatography step for the initial HCCF load with varying concentrations of HPBCD (Example section C-III).

**Description of Embodiments**

**Method for Viral inactivation**

The instant disclosure first relates to a method for inactivating an enveloped virus in a biopharmaceutical-containing fluid, said method comprising exposing said fluid to a substituted cyclodextrin.

The terms "biopharmaceutical-containing fluid" and "fluid comprising a biopharmaceutical" or "fluid containing a biopharmaceutical" or are used interchangeably throughout and have the same meaning.

By "exposing" it is meant that the biopharmaceutical-containing fluid is in contact or in presence of the substituted cyclodextrin. This may be performed by introducing the substituted cyclodextrin into the biopharmaceutical-containing fluid.

Viruses are classified as enveloped and non-enveloped viruses. Enveloped viruses have a capsid enclosed by a lipoprotein membrane or "envelope". This envelope is made up of host cell proteins and phospholipids as well as viral glycoproteins which coat the virus as it buds from its host cell. This envelope allows the virus to identify, bind, enter, and infect target host cells.

The enveloped viruses that are inactivated according to the disclosure may be selected from *Herpesviridae, Poxviridae, Hepadnaviridae, Flaviviridae, Togaviridae, Coronaviridae, Orthomyxoviridae, Delta virus, Paramyxoviridae, Rhabdoviridae, Bunyaviridae, Fdoviridae, Retroviridae* virus families, or from any combination thereof. It may for example be selected from the following species or from any combination thereof: human immunodeficiency virus, sindbis virus, herpes simplex virus, pseudorabies virus, sendai virus, vesicular stomatitis virus, West Nile virus, bovine viral diarrhea virus, a corona virus, equine arthritis virus, severe acute respiratory syndrome virus, Moloney murine leukemia virus, and vaccinia virus. It is more preferably selected from *Retroviridae* family, for example selected from *Alpharetrovirus, Betaretrovirus, Deltaretrovirus, Epsilonretrovirus, Gammaretrovirus, Lentivirus, Bovispumavirus, Equispumavirus, Felispumavirus, Prosimiispumavirus, Simiispumavirus* genera, or from any combination thereof. It is more preferably selected from *Gammaretrovirus* genus. It is for example Xenotropic murine leukemia virus (X-MuLV).

The terms "viral inactivation", "viral inactivation", "inactivate virus" or similar such phrases refer to a process where the enveloped virus is modified such that it can no longer infect cells, replicate, and/or propagate. To determine the extent or effectiveness of an agent, such as the substituted cyclodextrins described herein, to inactivate virus, one method is to monitor the impact on cytopathogenic effects (CPE). Cytopathic effects include structural changes in the host cell due to viral infection, such as host cell lysis, or cell death without lysis due to viral alterations that impact host cell division. If, after exposure to the agent, such effects in the host cell are not detectable, the virus is considered inactive. This may be measured using a Plaque assay which is used to determine the infectious titer of a virus which can cause cytopathic effects in a cell culture over a reasonable period, for example 5 to 20 days, while cells in culture remain viable. If active virus is present, the cytopathogenic effects on the cells can be observed using a microscope. Each infectious virus particle produces a circular zone of infected cells called a plaque. To determine the virus titer, the plaques are counted. The titer of a virus in each sample can be calculated in plaque-forming units (PFU) per milliliter, which can then be used to calculate the total log reduction of virus. Inactivation is considered complete if it is not detected at the limit of the detection method being used, typically about 4 log10. This ability to inactivate enveloped viruses may be determined by the person skilled in the art for example by measuring the level of infectivity of an enveloped virus such as X-MuLV, for example according to the protocol given in the Example section.

Preferably, according to this test, the cyclodextrin substitute according to the disclosure allows to inactivate a virus such that the total log reduction value (LRV) of the virus is at least of 4 log10.

Any degree of viral inactivation using the methods disclosed herein is desirable. However, it is preferred to achieve the degree of viral inactivation necessary to meet any safety guidelines or regulations for biopharmaceuticals as established by the relevant regulatory agency.

The expression « substituted cyclodextrin» classically refers to a mixture of cyclodextrin molecules, further comprising the residual substances resulting from its manufacturing process. Contrary to chemical substances with well-defined structure, substituted cyclodextrins generally are a mixture of substituted cyclodextrin molecules having different substitution patterns, and which are thus structurally different. It is understood that a substituted cyclodextrin is different from a cyclodextrin polymer, the latter being composed of cyclodextrin molecules covalently bound to one another.

Preferably, the substituted cyclodextrin is substituted with substituted with alkyl groups, hydroxyalkyl groups, or sulfoalkyl groups, preferably having 1 to 5 carbon atoms, preferably 1 to 4, preferably 1 to 3, preferably 1 or 3. Preferably, the substituted cyclodextrin according to the disclosure is substituted by etherification. It is preferably selected from hydroxypropyl-cyclodextrin, methyl-cyclodextrin, sulfobutyl ether cyclodextrin, or from a mixture thereof. It is preferably selected from hydroxypropyl-beta-cyclodextrin (HPBCD), methyl-beta-cyclodextrin, sulfobutyl-ether beta-cyclodextrin, or from a mixture thereof. It is preferably selected from hydroxypropyl-cyclodextrin, methyl-cyclodextrin, or from a mixture thereof. It is preferably selected from HPBCD, methyl-beta-cyclodextrin, or from a mixture thereof. It is preferably hydroxypropyl-cyclodextrin, more preferably HPBCD.

Preferably, the substituted cyclodextrin according to the disclosure, preferably HPBCD, has an average molar substitution degree (MS) equal to or higher than 0.10, preferably equal to or higher than 0.20, preferably equal to or higher than 0.30, preferably equal to or higher than 0.40, preferably equal to or higher than 0.50, preferably equal to or higher than 0.60, preferably equal to or higher than 0.70, preferably equal to or higher than 0.80, preferably equal to or higher than 0.81. Preferably, this MS is equal to or lower than 1.50, preferably equal to or lower than 1.40, preferably equal to or lower than 1.30, preferably equal to or lower than 1.20, preferably equal to or lower than 1.10, preferably equal to or lower than 1.00, preferably equal to or lower than 0.99. It is for example from 0.81 to 0.99.

It is reminded that the "average molar substitution degree (MS)" refers to the average number of added substituents per anhydroglucose unit. It should be noted that this MS is different from the average molecular substitution degree (DS), which refers to the average number of added substituents per cyclodextrin molecule, and which is, as a consequence, function of the number of anhydroglucose units forming the original cyclodextrin. For instance, for alkylated β-cyclodextrins, this DS is equal to 7 times the MS, as the β-cyclodextrins are made of 7 anhydroglucose units.

The MS of the hydroxypropyl-cyclodextrin according to the disclosure (e.g., HPBCD), can classically be determined by the person skilled in the art by proton nuclear magnetic resonance (NMR), preferably according to the USP 41 NF 36 method « Hydroxypropyl Betadex ; Molar substitution », as in force on January 1st, 2024.

Preferably, the HPBCD according to the disclosure has the following substitution profile, as determined by electrospray ionization - Mass spectrometry (ESI-MS):

- signal corresponding to unsubstituted cyclodextrin and hydroxypropyl-cyclodextrin molecules having a maximum of 3 substitutions (HP≤3): equal to or lower than 15%, preferably equal to or lower than 10%, preferably equal to or lower than 5%, preferably equal to or lower than 4%, preferably equal to or lower than 3%, preferably equal to or lower than 2%, preferably equal to or lower than 1%; and/or,
- signal corresponding to the hydroxypropyl-cyclodextrin molecules having 4 substitutions (HP4): equal to or lower than 25%, preferably equal to or lower than 20%, preferably equal to or lower than 15%, preferably equal to or lower than 10%, preferably equal to or lower than 5%, preferably equal to or lower than 4%, preferably equal to or lower than 3%, preferably equal to or lower than 2%, preferably equal to or lower than 1%; and/or,
- signal corresponding to the hydroxypropyl-cyclodextrin molecules having 5 substitutions (HP5): equal to or lower than 35%, preferably from 1 to 35%, preferably from 1 to 30%, preferably from 1 to 25%, preferably from 1 to 20%, preferably from 1 to 15%, preferably from 1 to 10%; and/or,
- signal corresponding to the hydroxypropyl-cyclodextrin molecules having 6 substitutions (HP6): from 1 to 40%, preferably from 1 to 30%, preferably from 1 to 25%, preferably from 1 to 20%, preferably from 5 to 20%, preferably from 5 to 15%; and/or,
- signal corresponding to the hydroxypropyl-cyclodextrin molecules having 7 substitutions (HP7): from 5 to 45%, preferably from 10 to 40%, preferably from 15 to 40%, preferably from 15 to 35%; and/or,
- signal corresponding to the hydroxypropyl-cyclodextrin molecules having 8 substitutions (HP8): from 1 to 45%, preferably from 5 to 40%, preferably from 10 to 40%, preferably from 15 to 40%, preferably from 20 to 35%; and/or,
- signal corresponding to the hydroxypropyl-cyclodextrin molecules having 9 substitutions (HP9): from 1 to 40%, preferably from 5 to 35%, preferably from 10 to 30%, preferably from 15 to 25%; and/or,
- signal corresponding to the hydroxypropyl-cyclodextrin molecules having 10 substitutions (HP10): equal to or lower than 20%, preferably equal to or lower than 15%, preferably from 1 to 15%, preferably from 5 to 15%; and/or,
- signal corresponding to the hydroxypropyl-cyclodextrin molecules having at least 11 substitutions (HP≥11): equal to or lower than 20%, preferably equal to or lower than 15%, preferably equal to or lower than 10%, preferably from 1 to 10%;

these percentages being expressed with respect of the sum of the signals obtained for each substitution for which the signal was higher than the background.

It is understood that "signal" refers to the area under the curve of the ion(s) corresponding to the substitutions degree(s) of interest.

This substitution profile is determined by ESI-MS preferably by calculating the average of the measurements performed in triplicate. For determining this substitution profile, it is possible to proceed according to the following method:

- A solution hydroxypropyl-cyclodextrin is prepared at 1 g.L-1 (w/v) in methanol: water (50/50, v/v) with 1 mM sodium acetate. Infusion is performed for 1 min at 10 μL/min and data are recorded. In between two following injections, 500 μL of methanol: water (50/50, v/v) are injected to wash the ion source.
- ESI parameters: Spray voltage: 5 kV; Sheath gas: 9; Auxiliary gas: 2; Sweep gas: 0; Spray voltage: 5 kV; Capillary voltage: 23 V; Capillary temperature: 275 °C; Tube lens: 80 V.
- MS parameters: Full scan; Scan ranges: 50-2000 m/z; Mass range: normal; Scan rate: enhanced; Data acquisition time: 1 min.
- For each substituted hydroxypropyl-cyclodextrin species (named as HPX, X being the number of hydroxypropyl substitutions), the corresponding ion current (XIC) is integrated and compared to the sum of all HPX ion currents. As the sodium adduct is the most intense hydroxypropyl-cyclodextrin ion (almost 100 times superior to [M+H]+ or [M+NH4]+), its peak area is integrated to estimate the proportion of the corresponding HPX molecule.

Preferably, the substitution pattern of the HPBCD according to the disclosure is such that:

- the molar proportion of unsubstituted (no-OHP) moieties is from 20 to 70%, preferably from 25 to 60%, preferably from 30 to 50% preferably from 30 to 40%; and/or,
- the molar proportion of C2 substituted (2 OHP) moieties is from 10 to 50%, preferably from 15 to 45%, preferably from 20 to 40%, preferably from 20 to 35%, preferably from 25 to 35%; and/or,
- the molar proportion of C3 substituted (3 OHP) moieties is from 2 to 15%, preferably from 4 to 10%, preferably from 6 to 8%; and/or,
- the molar proportion of C6 substituted (6 OHP) moieties is from 0.5 to 10%, preferably from 1 to 8%, preferably from 2 to 7% preferably from 3 to 6%, preferably from 4 to 6%; and/or,
- the molar proportion of C2 and C3 substituted (2,3-di-OHP) moieties is from 1 to 20%, preferably from 5 to 15%, preferably from 10 to 15%; and/or,
- the molar proportion of C2 and C6 substituted (2,6-di-OHP) moieties is from 0.5 to 15%, preferably from 1 to 10%, preferably from 2 to 9%, preferably from 3 to 8%, preferably from 4 to 7%; and/or,
- the molar proportion of twice C3 substituted (3,3'-di-OHP) moieties is equal to or lower than 10%, preferably equal to or lower than 5%, preferably equal to or lower than 3%, preferably equal to or lower than 2%, preferably equal to or lower than 1%; and/or,
- the molar proportion of C2, C3 and C6 substituted (2,3,6-tri-OHP) moieties is equal to or lower than 10%, preferably from 1 to 10%, preferably from 2 to 5%; and/or,
- the molar proportion of substitutions corresponding to mono-substitutions is from 40 to 90%, preferably from 50 to 85%, preferably from 60 to 80%; preferably from 60 to 70%; and/or,
- the molar proportion of substitutions corresponding to di-substitutions is from 5 to 50%, preferably from 10 to 45%, preferably from 15 to 40%, preferably from 20 to 40%, preferably from 25 to 40%, preferably from 30 to 35%; and/or,
- the molar proportion of substitutions corresponding to tri-substitutions is equal to or lower than 15%, preferably from 0.5 to 15%, preferably from 0.5 to 10%, preferably from 1 to 7%, preferably from 1 to 5%, preferably from 2 to 5%; and/or,
- the C2/C6 substitutions molar ratio is from 1.0 to 15.0, preferably from 1.5 to 12.0, preferably from 2.0 to 10.0, preferably from 2.5 to 9.0, preferably from 3.0 to 8.0, preferably from 3.0 to 5.0, preferably from 3.0 to 4.5, preferably from 3.5 to 4.5; and/or,
- the C2/C3 substitutions molar ratio is from 0.5 to 5.0, preferably from 1.0 to 4.0, preferably from 1.5 to 3.5, preferably from 2.0 to 3.0, preferably from 2.0 to 2.5.

The percentages correspond to the percentages of anhydroglucose units having the type of substitution considered. For example, a 2 OHP value equal to 30.0% means that 30.0 mol% of the anhydroglucose units of the hydroxypropyl-cyclodextrin are substituted by a hydroxypropyl group at the C2 carbon (and have not further substitutions). As a further example, a 3,3'-di-OHP value equal to 0.4% means that 0.4 mol% of the anhydroglucose units of the hydroxypropyl-cyclodextrin are substituted twice at the C3 carbon i.e., that the C3 carbon bears two hydroxypropyl groups (and the anhydroglucose units have not further substitutions). As a last example, a 2,6-di-OHP value equal to 5.0% means that 5.0 mol% of the anhydroglucose units of the hydroxypropyl-cyclodextrin are substituted by a hydroxypropyl group both at the C2 carbon, and at the C6 carbon (and have not further substitutions). For the first example, mention will be made of mono-substitution, whereas mention will be made of di-substitution for the last two examples.

Preferably, the hydroxypropyl-cyclodextrin according to the disclosure comprises less than 5.0% of substitutions other than those listed above, for example 3,6-OHP substitutions, preferably less than 4.0%, preferably less 3.0%, preferably less than 2.0%, preferably less than 1.0%, preferably less than 0.5%. Still preferably, the hydroxypropyl-cyclodextrin according to the disclosure comprises no other types of substitutions than those listed above. The expression "no other types of substitutions" is understood to mean that the anhydroglucose units comprising such substitutions are not detectable, in particular by the "Hakomori" method, said method comprising subjecting the hydroxypropyl-cyclodextrin to the following successive steps: permethylation, hydrolysis, reduction, peracetylation, analyzis by gas chromatography (GC) and gas chromatography coupled with mass spectrometry (GC-MS).

Indeed, these substitution patterns may be determined by those skilled in the art according to a method analogous to the "Hakomori" method, by subjecting the hydroxypropyl-cyclodextrin to the following successive steps: permethylation, hydrolysis, reduction, peracetylation, analyzis by GC and GC-MS.

It is possible for example to follow the method as described below:

60% NaH powder (35 mg) is washed with 2 mL of Heptane. After homogenization and centrifugation at 3000 rpm for 1 min, heptane is eliminated. This step is repeated 5 times, and then the NaH is dried under Nitrogen. Next, 200 $\mu$L of anhydrous dimethyl sulfoxide, 100 $\mu$L of test solution (2-3 mg of product in 1 mL of anhydrous dimethyl sulfoxide after a few minutes at 70 °C if necessary), and 100 $\mu$L of methyl iodide are added. After agitation for 10 minutes, the reaction is stopped in an ice bath. After 2 minutes, 2 mL of chloroform and 2 mL of water are added. After homogenization and centrifugation at 3000 rpm for 1 min, the aqueous phase is eliminated, and the chloroform phase is washed 5 times with water and then evaporated. The dry residue is dissolved in acetone (25 $\mu$L), and 0,5 mL of aqueous trifluoroacetic acid is added, then stored in a screw-cap tube at 100 °C for 4 hours, and completely evaporated under Nitrogen. The residue is dissolved in 1 mL of methanol and evaporated under nitrogen. This step is repeated 3 times. The residue is dissolved in 500 $\mu$L of sodium borohydride solution (20 mg/mL), and the solution is placed under gentle agitation at room temperature for two hours. The solution is acidified with an acetic acid/methanol mixture (1:9; 2 drops), and then completely evaporated under Nitrogen. Boric acid is evaporated by co-distillation with methanol (1 to 2 mL) (5 times). The dry residue is treated with acetic anhydride (0,5 mL) at 100 °C for 4 hours, then completely evaporated under Nitrogen. Next, 2 mL of chloroform and 2 mL of water are added. After homogenization and centrifugation at 3000 rpm for 1 min, the aqueous phase is eliminated and the chloroform phase is washed 6 times with water, and then evaporated under Nitrogen. The residue is dissolved in 200 $\mu$L to 400 $\mu$L of chloroform, then analyzed by gas chromatography (GC) and gas chromatography coupled with mass spectrometry (GC-MS). The GC is performed for example on a Scion 456 system with a flame ionization detector, using helium as carrier gas. The gas GC-MS is performed for example on a Scion 456-SQ system, using helium as carrier gas. A capillary column (length 30 m, inner diameter 0,32 mm, film thickness 0,25 $\mu$m) made of 100% Dimethylpolysiloxane fused silica, for example, Agilent DB-1 ref 123-1032, may be used used. The temperature is programmed as follows: 0 minute at 120 °C, -> 230 °C at 2 °C per minute, 230 °C for 1 minutes, -> 300 °C at 10 °C per minute, 300 °C for 2 minutes. The flow is at 1.7 mL/min in constant flow.

Such hydroxypropyl-cyclodextrin is commercially available. For example, mention can be made of KLEPTOSE® HP Biopharma (Roquette Frères).

Preferably, the substituted cyclodextrin according to the disclosure complies with the US monograph as in force on January 1st, 2024. Preferably, the substituted cyclodextrin according to the disclosure complies with the European monograph as in force on January 1st, 2024.

The term "biopharmaceutical" also often referred to as "large molecules" refers to active ingredients which are produced from living organisms or contains components of living organisms. They may be artificially created through a production system (e.g., mammalian cells, bacteria), or extracted from biological sources such as blood plasma. In general, they have a high molecular weight (as compared to standard active ingredient also often referred to as "small molecules"). Biopharmaceuticals according to the disclosure may be selected from therapeutic proteins, nucleic acids, vaccines, cells, viral vectors, or from any mixture thereof. Biopharmaceuticals are in general quite fragile, especially as compared to small molecules.

The biopharmaceutical according to the disclosure includes pharmaceutical and veterinary active ingredients. It is more preferably an active ingredient for treating a mammal, preferably a human.

Preferably, the biopharmaceutical according to the disclosure is a therapeutic protein. The term "protein" classically refers, in its broad meaning, to oligopeptides, peptides and proteins, regardless the way they are manufactured and regardless their number of subunits. They may be native proteins, recombinant proteins, or fusion proteins. They are also inclusive of modifications including, but not limited to, glycosylation, lipid attachment, sulfation, gamma-carboxylation of glutamic acid residues, hydroxylation and ADP-ribosylation.

Proteins of interest include, among other things, secreted proteins, non-secreted proteins, intracellular proteins or membrane-bound proteins. Proteins of interest can be produced by cell lines. The expressed protein(s) may be produced intracellularly or secreted into the culture medium from which it can be recovered and/or collected. Proteins of interest are proteins that exert a therapeutic effect, for example, by binding a target, including targets derived therefrom, targets related thereto, and modifications thereof.

Therapeutic proteins according to the disclosure may be selected for example from enzymes, cytokines, hormones, growth factors, plasmatic factors, vaccines, or antibodies (preferably monoclonal antibodies (mAbs)).

Proteins of interest may include "antigen-binding proteins". Antigen-binding protein refers to proteins that comprise an antigen-binding region or antigen-binding portion that has a strong affinity for another molecule to which it binds (antigen). Antigen-binding proteins include, but are not limited to, antibodies, peptibodies, antibody fragments, antibody derivatives, antibody analogs, fusion proteins (including single-chain variable fragments (scFvs) and double-chain (divalent) scFvs, muteins, xMAbs, bispecific T cell engagers (BiTE®), and chimeric antigen receptors (CARs or CAR-Ts) and T cell receptors (TCRs).

Preferably, the protein according to the disclosure is an antibody. The term "antibody" includes reference to both glycosylated and non-glycosylated immunoglobulins of any isotype or subclass or to an antigen-binding region thereof that competes with the intact antibody for specific binding. Unless otherwise specified, antibodies include human, humanized, chimeric, multi-specific, monoclonal, polyclonal, heterolgG, bispecific, and oligomers or antigen binding fragments thereof. Antibodies include the IgGI-, IgG2- IgG3- or IgG4-type. Also included are proteins having an antigen binding fragment or region such as Fab, Fab', F(ab')2, Fv, diabodies, Fd, dAb, maxibodies, single chain antibody molecules, single domain VHH, complementarity determining region (CDR) fragments, scFv, diabodies, triabodies, tetrabodies and polypeptides that contain at least a portion of an immunoglobulin that is sufficient to confer specific antigen binding to a target polypeptide.

Also included are human, humanized, and other antigen-binding proteins, such as human and humanized antibodies, that do not engender significantly deleterious immune responses when administered to a human.

Also included are modified proteins, such as are proteins modified chemically by a non-covalent bond, covalent bond, or both a covalent and non-covalent bond. Also included are proteins further comprising one or more post-translational modifications which may be made by cellular modification systems or modifications introduced ex vivo by enzymatic and/or chemical methods or introduced in other ways.

Proteins of interest may also include recombinant fusion proteins comprising, for example, a multimerization domain, such as a leucine zipper, a coiled coil, an Fc portion of an immunoglobulin, and the like. Also included are proteins comprising all or part of the amino acid sequences of differentiation antigens (referred to as CD proteins) or their ligands or proteins substantially similar to either of these.

Preferably, the antigen-binding protein or antibody according to the disclosure comprises a Fab region and a Fc region. More preferably, the antigen-binding protein or antibody according to the disclosure has two heavy chains and two light chains. Preferably, the heavy chains have 1 variable domain, and 3 constant domains. Preferably, the lights chains have 1 variable domain, and 1 constant domain. It is thus preferably an IgG antibody.

Preferably, the step of exposing the biopharmaceutical-containing fluid to the substituted cyclodextrin occurs at a substituted cyclodextrin concentration equal to or higher than 1% (as g of substituted cyclodextrin per 100 mL of said fluid, also referred to as "(w/v)"), preferably equal to or higher than 5% (w/v) more preferably higher than 5% (w/v), preferably equal to or higher than 6% (w/v), preferably equal to or higher than 7% (w/v), preferably equal to or higher than 8% (w/v), preferably equal to or higher than 9% (w/v), preferably equal to or higher than 10% (w/v). It is in general equal to or lower than 30% (w/v), preferably equal to or lower than 25% (w/v), preferably equal to or lower than 20% (w/v), preferably equal to or lower than 15% (w/v). This concentration is typically a concentration sufficient to cause inactivation of an (or any) enveloped virus. This concentration is typically the concentration of the substituted cyclodextrin into the fluid.

Preferably, the fluid according to the disclosure is exposed to the substituted cyclodextrin for at least 1 minute, preferably at least 5 minutes, preferably at least 10 minutes, preferably at least 15 minutes, preferably more than 15 minutes, preferably at least 20 minutes, preferably at least 30 minutes, preferably at least 40 minutes, preferably at least 50 minutes. It is in general exposed for no more than 36 hours, even no more than 24 hours, even no more than 12 hours, even no more than 6 hours, even no more than 4 hours, even no more than 2 hours, even no more than 60 minutes.

Preferably, exposure of the fluid to the substituted cyclodextrin occurs at a temperature sufficient to cause inactivation of an (or any) enveloped virus. Preferably, exposure of the fluid to the substituted cyclodextrin occurs at a temperature equal to or higher than 5°C, preferably equal to or higher than 10°C, preferably equal to or higher than 15°C, preferably equal to or higher than 20°C. Preferably, this temperature is equal to or lower than 40°C, preferably equal to or lower than 30°C. it is for example of 23 ± 2°C.

Preferably, exposure of the fluid to the substituted cyclodextrin occurs at a pH selected from 5 to 9, preferably from 6 to 8, preferably of from 6.5 to 7.5.

It is understood that when referring to the cyclodextrin exposure temperature and exposure pH, it is referred to the temperature and pH of the biopharmaceutical-containing fluid in which the cyclodextrin is included.

Typically, the biopharmaceutical-containing fluid is a fluid known or suspected to contain an enveloped virus. Typically, the fluid is a liquid.

Inactivation of an enveloped virus with the substituted cyclodextrin according to the disclosure (referred to as "cyclodextrin-based viral inactivation" in the instant disclosure) can take place at one or more steps of a biopharmaceutical manufacturing process, from biopharmaceutical harvest to formulation of the biopharmaceutical (i.e., in a form that is suitable for administration, eventually after reconstitution in an appropriate media). It can take place following harvest, clarification and prior to capturing, in particular prior to a unit operation comprising affinity chromatography; following capturing and prior to filtering and/or a polish chromatography unit operation(s); in between polish chromatography steps;

prior to a viral filtration step and/or a nanofiltration step and/or an ultrafiltration/diafiltration (UF/DF) step.

In an embodiment, the fluid is from an effluent stream, eluate, pool, storage or hold from a unit operation comprising harvest, clarification, capture, filtering, or chromatography.

The term "unit operation" refers to a functional step that is performed in a process for manufacturing and/or purifying a biopharmaceutical. For example, a unit operation can include steps such as, but not limited to, harvesting, capture, purifying, polishing, viral inactivation, virus filtering, and/or adjusting the concentration and formulation containing biopharmaceutical. Unit operations can also include steps where fluid is pooled, held, and/or stored, such as capture pools, following harvest, chromatography or filtration, and fluid in holding or storing vessels, such as such as following harvest. A single unit operation may be designed to accomplish multiple objectives in the same operation, such as harvest and viral inactivation or capture and viral inactivation.

In an embodiment, the fluid is an eluate collected from harvest or clarification, which preferably involve depth filtration. In a preferred embodiment, the fluid is a depth filtration eluate. In an embodiment, the fluid is eluate collected from affinity chromatography, ion exchange chromatography, multimodal chromatography, hydrophobic interaction chromatography or hydroxyapatite chromatography. Preferably, the fluid is eluate collected from depth filtration, or from capture chromatography. Preferably, said capture chromatography is affinity chromatography. Preferably, the affinity chromatography is selected from Protein A, Protein G, Protein A/G, or Protein L affinity chromatography.

In an embodiment, the fluid is a pool containing harvested cell culture fluid, eluate from clarification, eluate from affinity chromatography, eluate from ion exchange chromatography, eluate from multimodal chromatography, eluate from hydrophobic interaction chromatography or eluate from hydroxyapatite chromatography. Preferably, said chromatography is affinity chromatography. Preferably, the affinity chromatography is selected from Protein A, Protein G, Protein A/G, or Protein L affinity chromatography. It is more preferably affinity chromatography.

In an embodiment, the fluid is clarified harvested cell culture fluid.

In an embodiment, the fluid is a pool containing clarified harvested cell culture fluid, and capture chromatography eluate. Preferably, said capture chromatography is affinity chromatography, preferably selected from Protein A, Protein G, Protein A/G, or Protein L affinity chromatography. It is more preferably Protein A affinity chromatography.

Preferably, in the instant disclosure, the substituted cyclodextrin is used as a Triton X-100 substitute.

Therefore, preferably, the method for inactivating an enveloped virus using a substituted cyclodextrin according to the disclosure excludes the use of Triton X-100.

Preferably, said method is used in a process for manufacturing or purifying a biopharmaceutical, in particular in a biomanufacturing process i.e., in a manufacturing process that utilizes biological systems to produce biopharmaceuticals.

## Method for purifying / manufacturing a biopharmaceutical

The instant disclosure also relates to a method for purifying a biopharmaceutical comprising:

1. subjecting a biopharmaceutical-containing fluid through unit operations, said unit operations including:

- harvest and clarification,
- viral inactivation,
- capture,
- polish chromatography,
- filtering;

wherein at least one of a unit operation including viral inactivation comprises exposing the fluid to a substituted cyclodextrin;
and wherein harvest and clarification occur before a unit operation comprising capture;
and wherein capture occurs before a unit operation comprising polish chromatography and before a unit operation comprising filtering;

2. recovering a purified biopharmaceutical.

The instant disclosure also relates to a method for producing a purified biopharmaceutical comprising:

1. producing a biopharmaceutical-containing fluid;
2. processing said fluid through unit operations, said unit operations including:

- harvest and clarification,
- viral inactivation,

- capture,
- polish chromatography,
- filtering;

wherein at least one of a unit operation including viral inactivation comprises exposing the fluid to a substituted cyclodextrin;
and wherein harvest and clarification occur before a unit operation comprising capture;
and wherein capture occurs before a unit operation comprising polish chromatography and before a unit operation comprising filtering;

3. recovering a purified biopharmaceutical.

A single unit operation may be designed to accomplish multiple objectives in the same operation, such as harvest and viral inactivation or capture and viral inactivation. That is to say that in an embodiment, the unit operations include a unit operation comprising harvest and viral inactivation. In an embodiment, the the unit operations include a unit operation comprising capture and viral inactivation.

Preferably, the biopharmaceutical is as described in the claims and embodiments included in the present specification. Preferably, the fluid comprising the biopharmaceutical is as described in the claims and embodiments included in the present specification. Preferably, the substituted cyclodextrin is as described in the claims and embodiments included in the present specification. Preferably, the step of exposing the fluid to a substituted cyclodextrin as described before for the method for inactivating an enveloped virus according to the disclosure.

Preferably, the production of the biopharmaceutical-containing fluid comprises establishing a cell culture in a bioreactor. Preferably, the biopharmaceutical is a protein, and the production of the biopharmaceutical-containing fluid comprises establishing a cell culture in a bioreactor with a host cell expressing said protein.

"Cell" or "Cells" include any prokaryotic or eukaryotic cell. Cells can be either ex *vivo, in vitro* or *in vivo,* either separate or as part of a higher structure such as a tissue or organ. Cells include "host cells", also referred to as "cell lines", which are genetically engineered to express a protein of interest. Host cells are typically derived from a lineage arising from a primary culture that can be maintained in culture for an unlimited time. Genetically engineering the host cell involves transfecting, transforming or transducing the cells with a recombinant polynucleotide molecule, and/or otherwise altering (e.g., by homologous recombination and gene activation or fusion of a recombinant cell with a non-recombinant cell) to cause the host cell to express a desired recombinant protein. Methods and vectors for genetically engineering cells and/or cell lines to express a protein of interest are well known to those of skill in the art; for example, various techniques are illustrated in Current Protocols in Molecular Biology, Ausubel et al., eds. (Wiley & Sons, New York, 1990, and quarterly updates); Sambrook et al., Molecular Cloning: A Laboratory Manual (Cold Spring Laboratory Press, 1989); Kaufman, R. L, Large Scale Mammalian Cell Culture, 1990, pp. 15-69.A host cell can be any prokaryotic cell (for example, E. coli) or eukaryotic cell (for example, yeast, insect, or animal cells (e.g., CHO cells)). Vector DNA can be introduced into prokaryotic or eukaryotic cells via conventional transformation or transfection techniques.

Preferably, the cells according to the disclosure are host cells. A host cell, when cultured under appropriate conditions, expresses the protein of interest that can be subsequently collected from the culture medium (if the host cell secretes it into the medium) or directly from the host cell producing it (if it is not secreted). The selection of an appropriate host cell will depend upon various factors, such as desired expression levels, protein modifications that are desirable or necessary for activity (such as glycosylation or phosphorylation) and ease of folding into a biologically active molecule.

By "culture" or "culturing" is meant the growth and propagation of cells outside of a multicellular organism or tissue. Suitable culture conditions for mammalian cells are known in the art. Cell culture media and tissue culture media are interchangeably used to refer to media suitable for growth of cells during in vitro cell culture. Typically, cell culture media contains a buffer, salts, energy source, amino acids, vitamins and trace essential elements. Any media capable of supporting growth of the appropriate host cell in culture can be used.

Cells may be cultured in suspension or in an adherent form, attached to a solid substrate. Cell cultures can be established in fluidized bed bioreactors, hollow fiber bioreactors, roller bottles, shake flasks, or stirred tank bioreactors, with or without microcarriers.

Cell cultures can be operated in a batch, fed batch, continuous, semi-continuous, or perfusion mode. Mammalian cells, such as CHO cells, may be cultured in bioreactors at a small scale of less than 100 mL to less than 1000 mL. Alternatively, large scale bioreactors that contain 1000 mL to over 20,000 liters of media can be used. Large scale cell cultures, such as for clinical and/or commercial scale biomanufacturing of protein therapeutics, may be maintained for weeks and even months, while the cells produce the desired protein(s).

The purification and manufacturing methods according to the disclosure comprise unit operations including harvest and clarification, viral inactivation, capture, polish chromatography, filtering.

It may also include for example unit operations aiming at pooling, holding, and/or storing the fluid.

The unit operations include at least one unit operation comprising viral inactivation, wherein said viral inactivation comprises exposing the fluid to a substituted cyclodextrin (also referred to as "cyclodextrin-based viral inactivation" in the present disclosure).

Preferably, this viral inactivation is for inactivating an enveloped virus.

In the methods according to the disclosure, the unit operations may also include a unit operation comprising viral inactivation using other methods such as: heat inactivation/pasteurization; pH inactivation; UV and gamma ray irradiation; detergent inactivation using a compound other than the substituted cyclodextrin according to the disclosure; use of high intensity broad spectrum white light; addition of chemical inactivating agents such as B-propiolactone.

However, in order to preserve biopharmaceutical integrity, these additional methods are preferably not performed in the methods according to the disclosure.

Harvest and Clarification

In the methods according to the disclosure, there is a unit operation comprising harvest and clarification occurring before a unit operation comprising capture. The purpose of harvest and clarification is to collect and prepare the harvest material for downstream purification - in particular for the material to undergo capture, polishing, and filtering - by reducing the levels of impurities and particulates such as cell debris, media components, and other contaminants.

Methods for harvesting and clarifying biopharmaceuticals are known in the art. They include, but are not limited to, acid precipitation, accelerated sedimentation such as flocculation, separation using gravity, centrifugation, acoustic wave separation, filtration including membrane filtration using ultrafilters, microfilters, tangential flow filters, depth, and alluvial filtration filters. Therapeutic proteins expressed by prokaryotes may be retrieved from inclusion bodies in the cytoplasm by redox folding processes known in the art.

Preferably, harvesting and clarification comprises centrifugation, depth filtration, microfiltration, or a combination thereof.

Capture

In the methods according to the disclosure, there is at least one unit operation comprising capture, occurring after a unit operation comprising harvest and clarification, and occurring before a unit operation comprising polish chromatography, and before a unit operation comprising filtering. A unit operation comprising capture may occur immediately before a unit operation comprising polish chromatography, or immediately before a unit operation comprising filtering.

Capture may typically include capture chromatography that makes use of resins and/or membranes containing agents that will bind to the recombinant protein of interest, for example affinity chromatography, size exclusion chromatography, ion exchange chromatography, hydrophobic interaction chromatography (HIC), immobilized metal affinity chromatography (IMAC), and the like. Such materials are known in the art and are commercially available. Affinity chromatography according to the disclosure preferably includes a substrate-binding capture mechanism, an antibody- or antibody fragment-binding capture mechanism, an aptamer-binding capture mechanism, and a cofactor-binding capture mechanism, for example. Exemplary affinity chromatography includes a Protein A, Protein G, Protein A/G, or Protein L. A therapeutic protein of interest can be tagged with a polyhistidine tag and subsequently purified from IMAC using imidazole or an epitope, such a FLAG® and subsequently purified by using a specific antibody directed to such epitope.

Preferably, the unit operations of the methods according to the disclosure include a unit operation comprising capture chromatography, said chromatography being preferably selected from affinity chromatography, ion exchange chromatography, anion exchange chromatography, cation exchange chromatography, multi-modal chromatography, hydrophobic interaction chromatography, and hydroxyapatite chromatography. Preferably, said capture chromatography is affinity chromatography, preferably selected from Protein A, Protein G, Protein A/G, or Protein L affinity chromatography. It is even more preferably Protein A affinity chromatography.

Polishing and filtering

In the methods according to the disclosure, the unit operations include a unit operation comprising polish chromatography, performed after a unit operation comprising capture.

The term "polishing" or "polish chromatography" is used herein to refer to one or more chromatographic steps performed to remove remaining contaminants and impurities such as DNA, host cell proteins; product-specific impurities, variant products and aggregates and virus adsorption from a fluid including a biopharmaceutical that is close to a final desired purity. For example, polishing can be performed in bind and elute mode by passing a fluid including the biopharmaceutical through a chromatographic column(s) or membrane absorber(s) that selectively binds to either the target biopharmaceutical or the contaminants or impurities present in a fluid including the biopharmaceutical. In such an example, the eluate/filtrate of the chromatographic column(s) or membrane absorber(s) includes the biopharmaceutical.

The polish step preferably makes use of chromatography resins and/or membranes containing agents that can be used in either a flow-through mode (where the biopharmaceutical of interest is contained in the eluent that flows through the chromatography medium and the contaminants and impurities are bound to the chromatography medium) or bind and elute mode (where the biopharmaceutical of interest is bound to the chromatography medium and eluted from the chromatography medium after the contaminants and impurities have flowed through or been washed off the chromatography medium). Examples of such chromatography methods include ion exchange chromatography (IEX), such as anion exchange chromatography (AEX) and cation exchange chromatography (CEX); hydrophobic interaction chromatography (HIC); mixed modal or multimodal chromatography (MM), hydroxyapatite chromatography (HA); reverse phase chromatography and gel filtration.

Preferably, the polish chromatography according to the disclosure is selected from ion exchange chromatography, anion exchange chromatography, cation exchange chromatography, multi modal chromatography, hydrophobic interaction chromatography, and hydroxyapatite chromatography.

In the methods according to the disclosure, the unit operations include a unit operation comprising filtering. In general, the methods include more than one unit operations comprising filtering. At least one of those filtrations is performed after capture. Therefore, in the methods according to the disclosure, the unit operations include a unit operation comprising filtering, performed after a unit operation comprising capture.

Preferably, the filtering performed after capture is selected from virus filtration, depth filtration, nanofiltration, UF/DF.

Virus filtration can be performed using microfilters and/or nanofilters. Example of such filters are commercially available. Mention can be made for example of Plavona® (Asahi Kasei) and VPro® (EDM Millipore).

Preferably, filtering performed after capture includes at least one unit operation comprising nanofiltration and at least one unit operation comprising UF/DF. Preferably a unit operation comprising nanofiltration occurs prior to a unit operation comprising UF/DF. Filtering performed after capture can also include a unit operation comprising sterile filtration.

Typically, after capture, the methods according to the disclosure comprise more than one unit operations comprising polish chromatography, and more than one unit operations comprising filtering.

Cyclodextrin-based viral inactivation

Cyclodextrin-based viral inactivation according to the disclosure can be done at any time, and the methods according to the disclosure may include one or more unit operations comprising cyclodextrin-based viral inactivation.

Cyclodextrin-based viral inactivation according to the disclosure can take place as part of or after a harvest unit operation; prior to, as part of, or after one or more capture chromatography unit operations; prior to, as part of, or after one or more clarification unit operations; prior to, as part of, or after one or more affinity chromatography unit operations; prior to, as part of, or after one or more polish chromatography unit operations; prior to, as part of, or after one or more filtration unit operations.

It can take place prior to, as part of, or after one or more ion exchange chromatography, hydrophobic interaction chromatography, mixed modal or multimodal chromatography, and/or hydroxyapatite chromatography unit operations; prior to, as part of, or after one or more nanofiltration unit operations; and/or prior to or after one or more UF/DF unit operations.

In an embodiment a unit operation comprising cyclodextrin-based viral inactivation according to the disclosure occurs after a unit operation comprising harvesting. In an embodiment a unit operation comprising cyclodextrin-based viral inactivation according to the disclosure occurs before a unit operation comprising clarification. In an embodiment a unit operation comprising cyclodextrin-based viral inactivation according to the disclosure occurs after a unit operation comprising harvesting and before a unit operation comprising clarification.

In an embodiment a unit operation comprising cyclodextrin-based viral inactivation according to the disclosure occurs after a unit operation comprising clarification. In an embodiment a unit operation comprising cyclodextrin-based viral inactivation according to the disclosure occurs before a unit operation comprising capture.

In an embodiment a unit operation comprising cyclodextrin-based viral inactivation according to the disclosure occurs after a unit operation comprising capture. In an embodiment a unit operation comprising cyclodextrin-based viral inactivation according to the disclosure occurs before a unit operation comprising polish chromatography and/or before a unit operation comprising filtering.

In an embodiment a unit operation comprising cyclodextrin-based viral inactivation according to the disclosure occurs after a unit operation comprising polish chromatography. In an embodiment a unit operation comprising cyclodextrin-based viral inactivation according to the disclosure occurs after a unit operation comprising filtering.

In a particularly preferred embodiment, a unit operation comprising cyclodextrin-based viral inactivation according to the disclosure occurs after a unit operation comprising clarification and before a unit operation comprising capture.

In a particularly preferred embodiment, a unit operation comprising cyclodextrin-based viral inactivation according to the disclosure occurs after a unit operation comprising capture and before a unit operation comprising polish chromatography and/or before a unit operation comprising filtering, in particular before a unit operation comprising nanofiltration.

In particular, preferably, a unit operation including cyclodextrin-based viral inactivation occurs before a (or any) unit operation comprising UF/DF. In particular, preferably, a unit operation including cyclodextrin-based viral inactivation occurs before a (or any) unit operation comprising nanofiltration.

Preferably, the method for purifying or producing a purified biopharmaceutical according to the disclosure excludes the use of Triton X-100.

**Remarks**

The amounts of ingredients may be expressed in percentages by weight. These weights are amounts of ingredients as such, in their typical powdery or oily form (or liquid form for solvents). All compounds may include small amounts of impurities. Powdery ingredients may also include small amount of water (also referred to as %moisture or as "loss on drying").

Where an amount of ingredient in a product is selected in a range of from 0 to X% or expressed as being "less than X%" or "lower than X%", it is understood that an embodiment where said product is free of said ingredient is included.

Also, where a material is defined in generic terms (e.g., substituted cyclodextrin), in embodiments where the latter are selected among a list (from 1 to n species), said list can be used interchangeably with said generic term.

- For example, where the disclosure reads "in a preferred embodiment, the substituted cyclodextrin according to the disclosure is HPBCD", it is considered that the following disclosure "Preferably, the concentration of substituted cyclodextrin in the fluid is equal to or higher than 6%" also discloses the following embodiment: "Preferably, the concentration of HPBCD in the fluid is equal to or higher than 6%". This embodiment does not exclude the presence of other substituted cyclodextrin(s) in different amounts.

- As another example, where the specification states that the substituted cyclodextrin is "preferably selected from hydroxypropyl-cyclodextrin, methyl-cyclodextrin, or from a mixture thereof", it is considered that the following disclosure "Preferably, the concentration of substituted cyclodextrin in the fluid is equal to or higher than 6%" also discloses the following embodiment: "Preferably, the fluid includes a compound selected from hydroxypropyl-cyclodextrin, methyl-cyclodextrin, or from a mixture thereof, at a concentration equal to or higher than 6%". Again, this embodiment does not exclude the presence of other substituted cyclodextrin(s) in different amounts.

Still where a material is selected among a list, it is considered that embodiments where said material comprises or consist of said list are also disclosed. For example, where the disclosure reads "in a preferred embodiment, the substituted cyclodextrin according to the disclosure is HPBCD", the followings:

- "in a preferred embodiment, the substituted cyclodextrin according to the disclosure consists of HPBCD" and
- "in a preferred embodiment, the substituted cyclodextrin according to the disclosure comprises HPBCD"

are also considered to be disclosed.

It is understood that in the first case, HPBCD is the sole substituted cyclodextrin of the embodiment, while in the second case, other substituted cyclodextrins may be present.

It is reminded that, as used in the specification and in the claims, the term "comprising" may include the embodiments "consisting of" and "consisting essentially of". The terms "comprise(s)," "include(s)," "having," "has," "can," "contain(s)," and variants thereof, are intended to be open-ended transitional phrases that require the presence of the named feature(s)/element(s)/step(s)/unit operation(s) and permit the presence of other feature(s)/element(s)/step(s)/unit operation(s). However, such description should be construed as also describing products or methods "consisting of" and "consisting essentially of" the enumerated feature(s)/element(s)/step(s)/unit operation(s), which allows the presence of only the named feature(s)/element(s)/step(s)/unit operation(s), along with any impurities or moisture that might result therefrom, and excludes other feature(s)/element(s)/step(s)/unit operation(s).

It is reminded that, as used in the specification and in the claims, for describing in a method the order of steps/unit operations with one another, the terms "prior to", "before", "after", "followed by", "preceded by", may include "immediately before", or "immediately after". The terms "prior to", "before", "after", "followed by", "preceded by", and variants thereof, are intended to be open-ended transitional phrases that allow the presence of one or more other step(s)/unit operation(s) in between. However, such description should be construed as also describing steps/unit operations performed "immediately after" or "immediately before" one another, which does not allow the presence of any other step(s)/unit operation(s) in between.

It is reminded that, as used in the specification and the appended claims, the singular form "a," "an," and "the" comprise plural referents unless the context clearly indicates otherwise, and means "at least one" or "one or more". For example, reference to a component in the singular is intended to comprise a plurality of components. Thus, for example, "a" biopharmaceutical or "a" substituted cyclodextrin, means "one or more" biopharmaceutical or "one or more" substituted cyclodextrin, respectively.

It is reminded that, where the specification discloses more than one upper and lower limits for a numerical range, any combination of said upper and lower limits are also disclosed.

Other characteristics and advantages of the present invention will emerge clearly on reading the examples given hereinafter, which illustrate the invention without however limiting it.

**Examples**

**A- Viral inactivation efficiency of hydroxypropyl-beta cyclodextrin (HPBCD) on X-MuLV virus**

**A-I Introduction**

This study was designed to evaluate the viral inactivation efficiency of hydroxypropyl-beta-cyclodextrin (HPBCD) (KLEPTOSE® HP Biopharma, Roquette Frères). Identified process step samples were spiked with X-MuLV and checked for inactivation of the virus by analyzing samples on PG-4 cell lines. The effectiveness of these process steps on virus is indicated by the reduction in virus titer.

**A-II Materials and Methods**

**A-II, 1. Test items Details**

**Test Item-1:** Hydroxypropyl-beta-cyclodextrin (HPBCD): KLEPTOSE® HP Biopharma (Roquette Frères) ; Test Item Code : 232-0001 ; Storage Condition: 20-30°C.

A-II, 2. **Test Systems**

**Test System-1: Cell line**

- PG-4, ATCC No. CRL-2032.

**Test** System-2: **Virus**

- Enveloped model virus Xenotropic Murine Leukemia Virus (X-MuLV) ATCC No. VR-1447, whose properties are summarized in Table 1.

[Table 1] Properties of X-MuLV

| Virus | Family | Structure / genome | Size | PhysicoChemical resistance | Model/host |
|---|---|---|---|---|---|
| X-MuLV | *Retroviridae* | Enveloped, single stranded RNA | 70-100 nm | Low | Represents a nondefective C type retrovirus. Mandatory for biological products derived from CHO cell lines and monoclonal antibody products. |

**Test System-3: Infectivity assay**

- Plaque assay.

### A-II, 3. Test Items

Test items were prepared three times in 50 mM acetate buffer. HPBCD concentration in acetate buffer was of 10% (w/v) or 15% (w/v). The solutions thus obtained were filtered using 0.45 $\mu$m syringe filter (Poly ether sulfone membrane), and the reconstituted solution was stored at room temperature for 30 days.

The test item code, virus, run numbers, sample numbers, and description for each sample for the virus clearance study are given in Table 2.

[Table 2] Sample details ofVCS

| Test item code | Virus | Sample Number | Sample description |
|---|---|---|---|
| 232-0001 | X-MuLV | U-22271/001 | Load control (10%) |
| | | U-22271/002 | Processing control with 10% |
| | | U-22271/003 | Load control with 15% |
| | | U-22271/004 | Processing control with 15% |
| | | U-22271/005 | T=0 (10%) |
| | | U-22271/006 | T=60 (10%) |
| | | U-22271/007 | T=0 (15%) |
| | | U-22271/008 | T=60 (15%) |
| | | U-22271/009 | Loas control with media |
| | | U-22271/010 | Processing control with media |

### A-II, 4. Other Reagents

The followings other reagents were used in this study: 1M Tris Base (USP grade), 1M Acetic acid (USP grade), Water (Type 1 water), pH paper.

### A-II, 5. Media

The following media were used in this study:

- McCoy's 5A,
- McCoy's 5A with 10% FBS,
- McCoy's 5A with 10% FBS and 2 $\mu$g/mL polybrene.

### A-II, 6. Test Methods and Observations

A-II, 6.1. Preliminary study

*A-II, 6.1.1. Cell-based Cytotoxicity Evaluation*

The cells used in the cytotoxicity evaluation were exposed to a series of dilutions of the sample matrix solution for that process step. The cells were assessed for changes in morphology that could interfere with the evaluation of the indicator cells for viral cytopathic effects. The lowest dilution of the process sample solution showing no signs of cytotoxicity was determined and used for interference evaluation.

The process intermediates used in this study were tested for cytotoxicity on the PG-4 cells (used for titration of X-MuLV).

The samples were tested for cytotoxicity onto the PG-4 indicator cells.

The cytotoxicity sample's pH was adjusted to 6.79 (for HPBCD 10%) and 6.78 (for HPBCD 15%) with 1M Tris base. The test item was be filtered through 0.45 $\mu$m filter (PES) and were evaluated for cytotoxicity on PG-4 indicator cells in 6 well plate as duplicate. They were tested at undiluted, 2-fold, 5-fold, 10-fold, 20-fold and 50-fold dilutions using McCoy's 5A incomplete medium as diluent. Incomplete McCoy's 5A medium was used as cell control/negative control. Inoculation, incubation and observation were performed as follows:

1. PG 4 cells seeded in 6 well plates and incubated for 16-24 hours;
2. Media removed and sample added;
3. Incubate for adoption ;
4. Remove inoculum and replenish with media;
5. Incubate for 6-7 days;
6. Day 3/4 a media change;
7. Staining on 6/7th day;
8. Plaques counted microscopically/manually;
9. Calculate the titre and represent in terms of PFU/mL (for the interference evaluation).

The samples that showed >20% morphological changes in indicator cell line compared to negative control were considered as cytotoxic.

The noncytotoxic dilution of the process sample was used for interference evaluation.

The results of cytotoxicity are shown in Table 3, Example section A-III, 1.

A-II, 6.1.2. *Cell-based Interference Evaluation*

The interference evaluation was performed to test the possible interference of test samples on the assay test system used to titrate X-MuLV virus. The samples were tested for interference evaluation on PG-4 indicator cells.

The interference study samples pH was adjusted to pH 7.07 (for HPBCD 10%) and 6.95 (for HPBCD 15%) with 1M Tris base. The test items were then filtered through 0.45$\mu$m PES filter. Test items dilutions were prepared with incomplete McCoy's 5A media. Nontoxic and subsequent dilution (20-fold and 50-fold) of the test items were spiked with X-MuLV virus stock solution to achieve 5% (v/v) of the X-MuLV virus. Serial dilutions (10-fold) of the virus spiked test items were performed using 20-fold and 50-fold dilution of respective samples as the diluent.

In addition to this, McCoy's 5A was spiked with X-MuLV to achieve 5% (v/v). Serial dilutions (10-fold) of the virus spiked medium were performed using incomplete McCoy's 5A medium as the diluent. These samples served as the positive controls for the interference study. McCoy's 5A alone was used as negative control.

The virus spiked sample dilutions and controls were tested in three wells per dilution. The titration plates were incubated in CO2 incubator and observed on 7th day. Virus titer was calculated by counting plaques as PFU/mL.

Note: 5-fold and 10-fold dilutions of the samples showed interference hence tested in 20-fold and 50-fold dilutions.

The titers from the tested sample were compared to the positive control virus titer. The sample dilution (50-Fold) which did not show difference in virus titers by $\pm$0.5 log10 compared to positive control was considered as non-interference dilution. The lowest non-interference dilution (50-Fold) of the sample was considered for sample analysis.

The results of interference evaluation are shown in Table 4, Example section A-III, 2.

A-II, 6.2. Viral Clearance Study (VCS)

The virus stock solution was sonicated to remove any virus aggregates and filtered (0.45 $\mu$m PES) prior to spiking. All test samples were handled aseptically to avoid the introduction of contamination during assay performance. The pH adjustment was done using 1 M Tris.

The viral inactivation step was performed as single run. The evaluation was done under the following conditions:

- Temperature of operation : 23 $\pm$ 2°C,
- Volume of loading : 100 mL per run,
- pH for the step: 5.5,
- Virus : X-MuLV,
- Spike %: 5,
- HPBCD concentration: 10% and 15% (w/v),
- Mode of sample testing: Infectivity assay,
- Number of runs : 2 (1 run per HPBCD concentration),
- Incubation Time: 0 and 60 minutes.

A brief outline of the process step, and the samples collected and tested, is provided in Figure 1.

Test items and test item control were spiked with 5% of X-MuLV at pH 5.5 and approximately 7.00 respectively. Additionally, Mc Coys's 5A media also was spiked with 5% of X-MuLV at approximately 7.00, this served as media control.

Approximately 5 mL sample was removed after 0 min and 60 min incubation at 23 $\pm$ 2°C from test item, test item control and media control. All samples were filtered using 0.45 $\mu$m PES filters after adjusting/verifying the pH. pH papers were used to check the pH during VCS. 1 mL sample was used for infectivity assay. All back up samples generated during VCS were stored at -80 $\pm$ 5°C. Additional samples were discarded.

Results are shown in Figures 2 and 3.

A-II, 6.3. Validity Criteria

All assays met the acceptance criteria:

- Validity criteria for Cytotoxicity Assays: the negative controls did not show any evidence/effect of cytotoxicity.
- Validity criteria for Plaque assay: Negative control did not show any plaques. Stock Virus control titre value was within 95% confidence limit of the established virus titre. One last higher dilution showed no plaques.

A-II, 6.4. Calculation of Reduction Factors in Studies to Determine Viral Clearance

The Virus reduction factor was calculated using the following formula:

$$RF = \log [(C1 \times V1) / (C2 \times V2)]$$

Where:

RF: Reduction Factor
C1: Concentration of virus in the starting material
V1: Volume of the starting material
C2: Concentration of virus in the post processing material
V2: Volume of the post processing material

The reduction factor for the process step represents the logarithm of the ratio of the virus load at the beginning of the first process clearance step and at the end of the process clearance step. Reduction factor is normally expressed on a logarithmic scale.

A-II, 6.5. Statistical Analysis

The 95% confidence limits were calculated for virus titers and for all Log Reduction Value (LRV) calculations (wherever it is applicable). The details of statistical analysis are captured in Figures 2 and 3.

**A-III Results**

**A-III, 1. Cytotoxicity Results**

[Table 3] Cytotoxicity evaluation results

| Sample ID | Sample description | Non-Toxic dilution |
|---|---|---|
| 232-0001 | 10% HPBCD | 5-Fold |
| 232-0001 | 15% HPBCD | 5-Fold |
| Control | Negative control (McCoy's 5A) | Did not show any evidence/effect of cytotoxicity |

Lowest dilution with no evidence of cytotoxicity was considered as non - cytotoxic dilution. Nontoxic and subsequent dilution of samples were used for the interference study.

**A-III, 2. Interference Results**

[Table 4] Cell base interference evaluation results

| Sample ID | Sample description | Non-interference dilution | Result (Log PFU/mL) |
|---|---|---|---|
| 232-0001 | 10% HPBCD | 50-Fold | 5.19 |
| 232-0001 | 15% HPBCD | 50-Fold | 5.13 |
| Control | Negative control (McCoy's 5A) | NA | Did not show presence of virus |
| Control | Positive control (McCov's +X-MuLV) | NA | 5.60 |

Lowest sample dilution showing virus titers within $\pm 0.5$ log10 of positive control was considered and reported as non-interference dilution. The lowest non-interference dilution of the samples was considered for sample analysis.

### A-III, 3. Virus Clearance Study Results

Results are shown in Figure 2 and 3.
The calculation were the followings:

$$\text{Adjusted titer = Virus titer (PFU/mL) X pH adjust (if applicable) X Dilution adjust X Volume adjust}$$

Where:

$$\text{Virus Titer ((PFU/mL) = Virus titer/mL;}$$

-   Dilution adjust = non-interference dilution of the sample

Formula for Log Reduction Factor (RF) calculation: Example section A-II, 6.4.
Formula for 95% Conf. of Log10 Reduction:

$$\mp\sqrt{S2 + a^2}$$

where:

+ $S$ = 95% confidence limits for the viral assays of the starting material.
+ $a$ = 95% confidence limits for the viral assays for material after the step.

### A-III, 4. Log Reduction Factor Calculation

Overall log reduction factor for the process is tabulated in Table 5.

[Table 5] Overall Log10 Reduction Factor

| Test Item | Time Point | Sample No. | Condition | Log10 Reduction |
|---|---|---|---|---|
| HPBCD with 10% | T = 0 min | U-22271/005 | pH 5.5 | $\geq 5.13$ |
| HPBCD with 10% | T = 60 min | U-22271/006 | pH 5.5 | $\geq 5.13$ |
| HPBCD with 15% | T = 0 min | U-22271/007 | pH 5.5 | $\geq 5.13$ |
| HPBCD with 15% | T = 60 min | U-22271/008 | pH 5.5 | $\geq 5.13$ |

### A-IV Conclusion

This study was conducted to evaluate the viral inactivation efficiency of HPBCD. The process steps studied was effect of 10% and 15% of HPBCD on X-MuLV inactivation. Test item was incubated at two different concentration 10% and 15% at two different time points (T= 0 minutes and T=60 minutes).
The cumulative reduction (clearance) factor from the process steps demonstrated robust inactivation of the enveloped RNA virus X-MuLV. The clearance demonstrated was $\geq 5.13$ Log10 in 10% and 15% of HPBCD.

**B- Viral inactivation efficiency of HPBCD *versus* TNBP and Triton X-100 on X-MuLV virus, in mAb biomanufacturing process**

**B-I Introduction**

The aim of this study was to evaluate the viral inactivation efficiency of HPBCD (KLEPTOSE® HP Biopharma, Roquette Frères) and Tri-n-butyl phosphate (TNBP) & Triton X-100 in Clarified Harvest (Protein A Load) and Protein A eluate stage of monoclonal antibody (mAb) manufacturing process. Identified process step samples were spiked with X-MuLV, and check for inactivation of the virus by analyzing samples on PG-4 cell lines. The effectiveness of these process steps on virus is indicated by the reduction in virus titer.

**B-II Materials**

**B-II, 1. Test Items Details**

**Test item-1:**

- Hydroxypropyl-beta-cyclodextrin (HPBCD): KLEPTOSE® HP Biopharma (Roquette Frères) ;
- Test Item Code: 232-0001;
- Storage Condition: 20-30°C.

**Test item-2:**

- Clarified Harvest (Syngene International Limited);
- Storage Condition: Deep freezer/ULT (-50°C to -85°C);
- Protein Concentration: 1.63 mg/mL.

**Test Item-3:**

- Pro.A elute (Syngene International Limited);
- Storage Condition: Deep freezer/ULT (-50°C to -85°C);
- Protein Concentration: 12.5 mg/mL.

**Test Item-4:**

- Tri-n-butyl phosphate (Merck KGaA);
- Storage Condition: 20-30°C.

**Test Item-5:**

- Triton X-100 (Sigma Aldrich);
- Storage Condition: 20-30°C.

**B-II, 2. Test Systems**

**Test System-1: Cell line**

- PG-4, ATCC No. CRL-2032.

**Test System-2: Virus**

- Enveloped model virus Xenotropic Murine Leukemia Virus (X-MuLV) ATCC No. VR-1447, whose properties are summarized.

**Test System-3: Infectivity assays**

- TCID50 Assay;

- Plaque Assay.

**B-II, 3. Test Items**

B-II, 3.1. Preparation of HPBD

HPBCD was prepared two timed during the study.
Weighed 50 g of test item and dissolved in approximately 70 mL of 50 mM Acetate buffer and made up the volume to 100 mL with 50 mM Acetate buffer to get 50% solution. the solution was then filtered using 0.45 μm syringe filter (Poly ether sulfone membrane), and the reconstituted solution stored at room temperature for 30 days.

B-II, 3.2. Preparation of Test Item for Cytotoxicity interference and VCS

Test item for cytotoxicity interference and VCS are summarized in Table 6.

[Table 6] Test items for cytotoxicity interference and VCS

| Virus | VCS Run No. | VCS Sample Number | Sample description | Test item name |
|---|---|---|---|---|
| X-MuLV | 1 | U-23273/001 | Clarified harvest Load | Clarified harvest Load |
| | | U-23273/002 | Clarified harvest Processing Control | Clarified harvest Processing Control |
| | | U-23273/003 | Pro A Neutralized Elute Load | Pro A Elute Load |
| | | U-23273/004 | Pro A Neutralized Elute Processing Control | Pro A Elute Processing control |
| | | U-23273/005 | Clarified harvest + 5% HPBCD T=15 minutes | Clarified Harvest + 5% HPBCD |
| | | U-23273/006 | Clarified harvest + 5% HPBCD T=60 minutes | |
| | | U-23273/007 | Pro A Neutralized Elute + 5% HPBCD T=15 minutes | Pro A Elute + 5% HPBCD |
| | | U-23273/008 | Pro A Neutralized Elute + 5% HPBCD T=60 minutes | |
| | | U-23273/009 | Clarified harvest + 10% HPBCD T=15 minutes | Clarified Harvest + 10% HPBCD |
| | | U-23273/010 | Clarified harvest + 10% HPBCD T=60 minutes | |
| | | U-23273/011 | Pro A Neutralized Elute + 10% HPBCD T=15 minutes | Pro A Elute + 10% HPBCD |
| | | U-23273/012 | Pro A Neutralized Elute + 10% HPBCD T=60 minutes | |
| | | U-23273/013 | Clarified harvest+ 0.3%TNBP + 1 %Triton-X T=15 minutes | Clarified Harvest + 0.3% TNBP + 1% Triton X-100 |
| | | U-23273/014 | Clarified harvest + 0.3%TNBP + 1%Triton-X T=60 minutes | |
| | | U-23273/015 | Pro A Neutralized Elute + 0.3% TNBP + 1%Triton-X T=15 minutes | Pro A Elute + 0.3% TNBP + 1% Triton X-100 |

(continued)

| Virus | VCS Run No. | VCS Sample Number | Sample description | Test item name |
|---|---|---|---|---|
| | | U-23273/016 | Pro A Neutralized Elute + 0.3% TNBP + 1%Triton-X T=60 minutes | |
| | | U-23273/017 | Clarified harvest Load | Clarified harvest Load |
| | | U-23273/018 | Clarified harvest Processing Control | Clarified harvest Processing Control |
| | | U-23273/019 | Pro A Neutralized Elute Load | Pro A Elute Load |
| | | U-23273/020 | Pro A Neutralized Elute Processing Control | Pro A Elute Processing Control |
| | | U-23273/021 | Clarified harvest + 5% HPBCD T=15 minutes | Clarified Harvest + 5% HPBCD |
| | | U-23273/022 | Clarified harvest + 5% HPBCD T=60 minutes | |
| | 2 | U-23273/023 | Pro A Neutralized Elute + 5% HPBCD T=15 minutes | Pro A Elute + 5% HPBCD |
| | | U-23273/024 | Pro A Neutralized Elute+ 5% HPBCD T=60 minutes | |
| | | U-23273/025 | Clarified harvest + 10% HPBCD T=15 minutes | Clarified Harvest + 10% HPBCD |
| | | U-23273/026 | Clarified harvest + 10% HPBCD T=60 minutes | |
| | | U-23273/027 | Pro A Neutralized Elute + 10% HPBCD T=15 minutes | Pro A Elute + 10% HPBCD |
| | | U-23273/028 | Pro A Neutralized Elute+ 10% HPBCD T=60 minutes | |

## B-II, 4. Other Reagents

The other reagent/materials used were the followings: 1M Tris Base (USP grade), 1M Acetic acid (USP grade), Water (Type 1 water), pH paper.

Reagents Preparation: the reagents such as 1M Acetic Acid, 1M Tris base and 50 mM acetate buffer were prepared.

## B-II, 5. Media

The following media were used in this study:

-   McCoy's 5A,

-   McCoy's 5A with 10% FBS,

-   McCoy's 5A with 10% FBS and 2 μg/mL polybrene.

## B-II, 6. Test Methods, Observations and Results

B-II, 6.1. Preliminary Study

B-II, 6.1.1. Cell-based Cytotoxicity Evaluation

In the cytotoxicity evaluation, the cells used in the viral quantitation assay were exposed to a series of dilutions of the sample matrix. The cells were assessed for changes in morphology that could interfere with the evaluation of the indicator cells for viral cytopathic effects or plaque formation. The lowest dilution of the process sample solution showed no signs of cytotoxicity were determined and used for interference evaluation.

The test items were tested for cytotoxicity on the PG-4 cells. The cytotoxicity sample pH was adjusted to 6.50-7.50 with Acetic Acid.

The test items were filtered through 0.45 μm filter.

The test items were evaluated for cytotoxicity on PG-4 indicator cells in 6 well plates as duplicate.

The test items were tested at Undiluted, 2-fold, 5-fold, 10-fold, 20-fold and 50-fold dilutions using McCoy's 5A incomplete medium as diluent. Samples 9 and 12 were tested at 50-fold, 100-fold, 200-fold, 400 and 500-fold dilutions as Samples 3 and 6 showed cytotoxicity till 50-fold.

Incomplete McCoy's 5A medium was used as cell control/negative controls.

Inoculation, incubation, and observation were done as described before in section A-II, 6.1.1.

Interpretation of results: The samples that showed >20% morphological changes in indicator cell line compared to negative control are considered as cytotoxic. Lowest dilution with no evidence of cytotoxicity was considered as non - toxic dilution.

The noncytotoxic dilution of the process sample were used for interference evaluation.

Results of cytotoxicity assay are summarized in Table 7.

[Table 7] Cytotoxicity Results

| Test item Name | Non-Cytotoxic dilution |
|---|---|
| Clarified Harvest | Undiluted |
| Pro A Elute | Undiluted |
| Clarified Harvest + 10% HPBCD | Undiluted |
| Clarified Harvest + 5% HPBCD | Undiluted |
| Pro A Elute + 10% HPBCD | Undiluted |
| Pro A Elute + 5% HPBCD | Undiluted |
| Clarified Harvest + 0.3%TNBP +1% Triton X-100 | 100-Fold |
| Pro A Elute + 0.3%TNBP +1% Triton X-100 | 200-Fold |
| Control* | No Plaque Observed |
| | No Plaque Observed |
| | No Plaque Observed |
| * Three analyses performed hence three separate controls used. | |

B-II, 6.1.2. *Cell based Interference Evaluation*

The interference evaluation was performed to test the possible interference of test items on the assay test system used to titrate X-MuLV. The test item were tested for interference evaluation.

The interference study sample's pH was adjusted to pH 6.50-7.50 with Acetic Acid.

The test items were then filtered through 0.45μm.

The test items dilutions were prepared with incomplete McCoy's 5A media.

Noncytotoxic and subsequent 4 dilution of the test items were spiked with X-MuLV, virus stock solution to achieve 5% (v/v) of the X-MuLV, virus. However, Noncytotoxic and subsequent one dilution alone was tested for Protein A load and Protein A elute (Clarified Harvest and Pro A Neutralized Elute).

Spiking concentration used was 5%

Serial dilutions (10-fold) of the virus spiked test items/media were performed using the noncytotoxic and subsequent 4 dilutions of respective samples and media as the diluent.

Virus spiked in incomplete media served as the positive controls for the interference study McCoy's 5A alone served as negative control.

The virus spiked sample dilutions and controls were tested in three wells per dilutions.

Inoculation, incubation and observation were performed, as described above.

Interpretation of results: The titers from the tested sample were compared to the positive control virus titer. The samples dilution which does not show difference in virus titers by $\pm 0.5$ log10 compared to positive control were considered as non-interference dilution. The lowest non-interference dilution of the sample was considered for sample analysis during VCS.

Results of interference assay is summarized in Table 8.

[Table 8] Cell based interference evaluation results

| Test item Name | Non-interference dilution (Fold) | Titer (Log PFU/mL) |
|---|---|---|
| Clarified Harvest | Undiluted | 5.72 |
| Pro A Elute | Undiluted | 5.78 |
| Clarified Harvest + 5% HPBCD | 10 | 5.82 |
| Clarified Harvest + 10% HPBCD | 10 | 5.92 |
| Clarified Harvest + 0.3%TNBP + 1% Triton X-100 | 200-Fold | 5.56 |
| Pro A Elute + 5% HPBCD | 10 | 5.93 |
| Pro A Elute + 10% HPBCD | 10 | 5.99 |
| Pro A Elute + 0.3%TNBP +1% Triton X-100 | 200-Fold | 5.62 |
| Media spike* | Plaque Observed | 5.90 |
| | Plaque Observed | 5.56 |
| *Two separate assays were performed hence two media spikes used. #Not used for VCS. | | |

B-II, 6.2. Viral Clearance Study (VCS)

The virus stock solution was sonicated to remove any virus aggregates and filtered (0.45 μm PES) prior to spiking.

All test samples were handled aseptically to avoid the introduction of contamination during assay performance.

**Log Reduction Factor Calculation:**

The Virus reduction factor for each of the removal steps was calculated using the following formula:

$$RF = \log\left[(C1 \times V1) / (C2 \times V2)\right]$$

Where:

RF: Reduction Factor
C1: Concentration of virus in the starting material
V1: Volume of the starting material
C2: Concentration of virus in the post processing material
V2: Volume of the post processing material

The reduction factor for the process step represents the logarithm of the ratio of the virus load at the beginning of the clearance step and at the end of the process clearance step. Reduction factor is expressed on a logarithmic scale.

Lowest LRV (Log Reduction Value) obtained among duplicate runs for a step was used for the overall LRV calculation. LRV for each run was calculated and captured in the respective result tables for each run.

**Log10 Reduction:** Log10 Adjusted Titer (PFU) of starting material - Log10 Adjusted Titer (PFU) of post processing material.

**Formula for 95% Conf. of Log10 Reduction:**

$$\pm \sqrt{(S^2 + a^2)}$$

Where:

+S = 95% confidence limits for the viral assays of the starting material.

+a= 95% confidence limits for the viral assays for material after the step.

**Calculation for X-MuLV:** Virus Titre (PFU/mL) Value of 2.00 denotes limit of detection of the assay in case of Normal titration.

Limit of detection (per sample) = - [ln (0.05)/number of wells plated] x (dilution/volume plated per well)

= - [ln (0.05)/3] *(1/0.5)

= 2.00

Virus Titer (PFU/mL) Value of 0.10 denotes limit of detection of the assay in case of Large Volume Plating.

Limit of detection (per sample) = - [ln (0.05)/number of wells plated] x (dilution/volume plated per well)

= - [ln (0.05)/60] *(1/0.5).

= 0.10

**Description of Title used in the calculation**

Virus Titer (PFU/mL) = Virus titer/mL;
Dilution adjust = non -interference dilution of the sample
Volume adjust =Volume of starting material or post processing material Adjusted titer = Virus titer (PFU/mL) X pH adjust (if applicable) X Dilution adjust X Volume adjust Log10 adjusted Viral Titer = Log10 value of adjusted titer Log Reduction= Reduction Factor (RF)

**Statistical Analysis**

The 95% confidence limits were calculated for virus titers and for all Log Reduction Value (LRV) calculations. The results are captured in the individual tables.
The viral inactivation step was performed as duplicate run with HPBCD, whereas single run was performed for TNBP + Triton X-100 combination.

The evaluation was done under the following conditions:

**Viral inactivation using HPBCD:**

- Temperature of operation : 23 ± 2°C,
- Volume of loading : 50 mL per run,
- pH for the step: 6.5-7.5,
- Virus: X-MuLV,
- Spike %: 5,
- HPBCD Concentration: 10 % and 5%
- Mode of sample testing: Infectivity assay,
- Number of runs: 2,
- Incubation Time: 15 and 60 minutes,

**Viral inactivation using 0.3%TNBP + 1%Triton X-100**

- Temperature of operation: 23 $\pm$ 2° C,
- Volume of loading: 50 mL per run,
- pH for the step: 6.5-7.5,
- Virus: X-MuLV,
- Spike %: 5,
- TNBP Concentration: 0.3%,
- Triton-X 100 Concentration: 1%,
- Mode of sample testing: Infectivity assay,
- Number of runs: 1,
- Incubation Time: 15 and 60 minutes.

Two runs were performed for test items with HPBCD 5% and HPBCD 10%. However, one run alone performed for 0.3%TNBP + 1%Triton-X 100.

A brief outline of the process steps is provided in Figure 4.

A portion of each sample was tested immediately for infectivity assay.
Large Volume plating was performed with 10 six well plates for both time points (15 min and 60 min).

All back up samples were aliquoted and stored.

Results are summarized in Figures 5 to 14.

**Overall Log10 Reduction Factor:**

This section summarizes the overall LRV and cumulative Log10 Reduction factor. Table 9 describes about the overall lower LRV for each samples tested and Figures 15 and 16 detail the LRV of each test sample (i.e. 5%, 10% HPBCD and 0.3%TNBP +1% Triton-X) used in Clarified Harvest fluid or Protein A Eluate.

[Table 9] Overall Log10 Reduction Value forX-MuLV

| Test item Name | Time (min) | Run No. | $Log_{10}$ Reduction | Lower LRV (Log Reduction Value) |
|---|---|---|---|---|
| Clarified Harvest + 5% HPBCD | 15 | Run 1 | 1.71 | 1.62 |
| | | Run 2 | 1.62 | |
| | 60 | Run 1 | 1.78 | 1.74 |
| | | Run 2 | 1.74 | |
| Clarified Harvest + 10% HPBCD | 15 | Run 1 | 3.23 | 2.92 |
| | | Run 2 | 2.92 | |
| | 60 | Run 1 | 5.36 | 5.24 |
| | | Run 2 | 5.24 | |
| Pro A Neutralized Elute + 5% HPBCD | 15 | Run 1 | 1.80 | 1.66 |
| | | Run 2 | 1.66 | |
| | 60 | Run 1 | 1.85 | 1.85 |
| | | Run 2 | 2.03 | |

(continued)

| Test item Name | Time (min) | Run No. | Log$_{10}$ Reduction | Lower LRV (Log Reduction Value) |
|---|---|---|---|---|
| Pro A Neutralized + 10% HPBCD | 15 | Run 1 | 3.15 | 2.82 |
| | | Run 2 | 2.82 | |
| | 60 | Run 1 | 5.45 | 5.33 |
| | | Run 2 | 5.33 | |
| Clarified Harvest + 0.3 % TNBP + 1% Triton X | 15 | Run 1 | 4.06 | 4.06 |
| | 60 | | 4.06 | 4.06 |
| Pro A Neutralized + 0.3 % TNBP + 1% Triton X | 15 | Run 1 | 4.15 | 4.15 |
| | 60 | | 4.15 | 4.15 |

B-II, 6.3. Discussions

The study was performed to evaluate the viral inactivation efficiency of HPBCD and TNBP & Triton X-100 in Clarified Harvest (Protein A Load) and Protein A elute stage of monoclonal antibody manufacturing process. Two different concentrations of HPBCD (5% and 10%) were tested in this study with 15 minutes and 60 minutes duration against the enveloped virus X-MuLV. Two runs were performed in this study for each concentration and time points. Controls such as load and processing controls were also tested without HPBCD along with test samples. One run was performed with 0.3 % TNBP + 1% Triton X-100 in both Clarified Harvest (Protein A Load) and Protein A elute to compare the log10 reduction. All samples and controls were tested by cell-based infectivity assay using PG4 (S+L-) cell lines and the virus titer were reported as Plaque Forming Units. Log10 reduction was calculated comparing with Load value in each time point and concentration. Test item spiked with MuLV and 5 % HPBCD incubated at 15 minutes and 60 minutes showed less inactivation. Clarified harvest and protein A elute with 10 % of HPBCD when incubated for 60 minutes showed the highest viral inactivation than compared to 15 minutes incubation. The LRV was calculated as 5.24 and 5.33 in Clarified Harvest and protein A eluate, respectively. This indicates the robust inactivation of enveloped viruses. Additionally, clarified harvest and protein A elute with 0.3 % TNBP + 1% Triton X showed the LRV of 4.06 and 4.15 in both incubation period (15 &60 minutes).

## B-III Conclusion

The study was performed to evaluate the viral inactivation efficiency of HPBCD against TNBP & Triton-X combination in Clarified Harvest (Protein A Load) and Protein A elute stage of monoclonal antibody manufacturing process. Enveloped virus (X-MuLV) used in this study as the choice of virus. Test items (clarified Harvest and Protein A elute) were spiked with 5% of X-MuLV were incubated at 15 minutes and 60 minutes. These samples were tested along with Load and processing controls (Media spiked with virus HPBCD and TNBP & Triton X-100) by cell-based infectivity. The virus titers were quantified and log10 reduction Factors were calculated statistically. HPBCD in 10% concentration with 60 minutes showed very effective inactivation of enveloped virus than 5% concentrations and 15 minutes incubation. Inactivation was gradual and complete over the period of 60 minutes. As compared with the combination of 0.3 % TNBP + 1% Triton X was sudden in 15 minutes. Thus, HPBCD in 10% concentration with 60 minutes demonstrated the capability to inactivate enveloped viruses during product manufacturing and can be used as a viral inactivating agent in biomanufacturing.

## C- Compatibility of HPBCD in downstream processes

While HPBCD showed effective virus inactivation in downstream processing, its compatibility with the downstream processing steps needed to be evaluated. Ideally, the spiking of HPBCD in the processing intermediate does not impact the product stability, product yield, and impurity removal efficiency of the target biopharmaceutical.

## C-I Materials

Hitrap MabSelect PrismA (5 mL), HiTrap Q HP (5 mL), and HiTrap SP HP (5 mL) columns were from Cytiva. Ipilimumab cell culture was produced in-house using the stable Chinese hamster ovary (CHO) cell line. All chromatography was run on the AKTA Pure 25 M system. Host cell protein (HCP) ELISA kit (ab240996) and protein A ELISA kit (ab133036) were purchased from Abcam. Host cell DNA (HCDNA) Q-PCR kit (OPA-R004) was obtained from ACROBiosystems.

## C-II Methods

The samples loaded onto protein A chromatography were prepared by spiking the harvested cell culture fluid (HCCF) of ipilimumab with different concentrations of HPBCD (KLEPTOSE® HP Biopharma, Roquette Frères) (Table 10). 100 mL of each sample was loaded onto Hitrap MabSelect PrismA and eluted with 50 mM acetic acid. The neutralized protein A eluate was further purified by anion exchange chromatography (AIEX) using HiTrap Q HP column with flow-through mode, and cation exchange chromatography (CIEX) using HiTrap SP HP column with bind-elute mode. The chromatography parameters were the same for the samples spiked with 0%, 5%, and 10% HPBCD. Each sample was purified in duplicate. To understand the stability of ipilimumab in terms of size and charge profile, the protein A eluate, AIEX flowthrough (FT), and CIEX eluate were analyzed by size exclusion chromatography (SEC-HPLC) and cation exchange chromatography (CIEX-HPLC). The impurity of HCP and HCDNA in the same samples were measured using a correspondent ELISA kit and Q-PCR assay following the manufacturer's protocols, respectively. The leaked protein A was measured using a protein A ELISA kit.

[Table 10] Spike of HPBCD into harvested cell culture fluid (HCCF) for downstream processes

| Sample | HCCF | 40% HPBCD stock | Binding buffer | Total volume |
|---|---|---|---|---|
| HCCF | 180 mL | 0 mL | 60 mL | 240 mL |
| HCCF + 5% HPBCD | 180 mL | 30 mL | 30 mL | 240 mL |
| HCCF + 10% HPBCD | 180 mL | 60 mL | 0 mL | 240 mL |

## C-III Results

Spiking of HPBCD in HCCF did not affect the protein stability in downstream process steps. The adalimumab collected from each chromatography step had comparable size (Table 11) and charge (Table 12) profiles, despite that the starting HCCF was spiked with 0%, 5%, or 10% HPBCD.

HPBCD did not have negative impact on host cell protein (HCP) removal efficiency (Figure 17). All samples had comparable level of HCP after each column chromatography. There was no detectable HCP after anion exchange chromatography for all samples. Similar results were observed for host cell DNA removal (Figure 18) and residual protein A removal (Figure 19). The results indicate that HPBCD is compatible with the downstream processes. The product stability and impurity removal efficiency are not affected by spiking HPBCD into the purification intermediates.

[Table 11] Size profile of purified adalimumab samples with different starting HPBCD concentrations

| Sample | protein A Eluate | | | AIEX Flowthrough | | | CIEX Eluate | | |
|---|---|---|---|---|---|---|---|---|---|
| | HMW | Main Peak | LMW | HMW | Main Peak | LMW | HMW | Main Peak | LMW |
| HCCF | 0.50 ± 0.11 | 99.42 ± 0.01 | 0.08 ± 0.08 | 0.46 ± 0.03 | 99.54 ± 0.03 | 0 | 0.57 ± 0.04 | 99.43 ± 0.04 | 0 |
| HCCF + 5% HPBCD | 0.43 ± 0 | 99.41 ± 0 | 0.16 ± 0 | 0.49 ± 0.01 | 99.51 ± 0.03 | 0 | 0.61 ± 0.01 | 99.39 ± 0.01 | 0 |
| HCCF + 10% HPBCD | 0.43 ± 0 | 99.42 ± 0 | 0.15 ± 0 | 0.48 ± 0.01 | 99.52 ± 0.03 | 0 | 0.61 ± 0.01 | 99.39 ± 0.01 | 0 |

[Table 12] Charge profile of purified adalimumab samples with different starting HPBCD concentrations

| Sample | protein A Eluate | | | AIEX Flowthrough | | | CIEX Eluate | | |
|---|---|---|---|---|---|---|---|---|---|
| | Acidic | Main | Basic | Acidic | Main | Basic | Acidic | Main | Basic |
| HCCF | 31.64 ± 1.08 | 59.52 ± 0.84 | 8.85 ± 0.25 | 27.74 ± 0.23 | 60.51 ± 0.28 | 11.75 ± 0.05 | 28.49 ± 0.20 | 60.14 ± 0.29 | 11.38 ± 0.10 |

(continued)

| Sample | protein A Eluate | | | AIEX Flowthrough | | | CIEX Eluate | | |
|---|---|---|---|---|---|---|---|---|---|
| | Acidic | Main | Basic | Acidic | Main | Basic | Acidic | Main | Basic |
| HCCF +5% HPBCD | 33.48 ± 0.52 | 57.68 ± 0.74 | 8.84 ± 0.21 | 29.52 ± 0.78 | 59.44 ± 0.69 | 11.05 ± 0.10 | 29.93 ± 0.05 | 58.56 ± 0.23 | 11.52 ± 0.19 |
| HCCF +10% HPBCD | 34.74 ± 1.02 | 56.17 ± 0.72 | 9.09 ± 0.30 | 31.40 ± 0.57 | 57.39 ± 0.64 | 11.22 ± 0.07 | 31.61 ± 0.82 | 56.18 ± 0.91 | 12.22 ± 0.09 |

**Claims**

1. A method for inactivating an enveloped virus in a biopharmaceutical-containing fluid, said method comprising exposing said fluid to a substituted cyclodextrin.

2. The method of claim 1 wherein said exposure occurs at a substituted cyclodextrin concentration equal to or higher than 1% (w/v).

3. The method of any of claims 1 or 2 wherein said biopharmaceutical-containing fluid is exposed to said substituted cyclodextrin for at least 1 minute.

4. The method of any of claims 1 to 3 wherein exposure of said biopharmaceutical-containing fluid to said substituted cyclodextrin occurs at a temperature higher than 5°C and lower than 50°C.

5. The method of any of claims 1 to 4 wherein exposure of said biopharmaceutical-containing fluid to said substituted cyclodextrin occurs at pH selected from 5 to 9.

6. A method for purifying a biopharmaceutical comprising:

   1. subjecting a biopharmaceutical-containing fluid through unit operations, said unit operations including:

      - harvest and clarification,
      - viral inactivation,
      - capture,
      - polish chromatography,
      - filtering;
      wherein at least one of a unit operation including viral inactivation comprises exposing the fluid to a substituted cyclodextrin;
      and wherein harvest and clarification occur before a unit operation comprising capture;
      and wherein capture occurs before a unit operation comprising polish chromatography and before a unit operation comprising filtering;

   2. recovering a purified biopharmaceutical.

7. A method for producing a purified biopharmaceutical comprising:

   1. producing a biopharmaceutical-containing fluid;
   2. processing said fluid through unit operations, said unit operations including:

      - harvest and clarification,
      - viral inactivation,
      - capture,
      - polish chromatography,
      - filtering;
      wherein at least one of a unit operation including viral inactivation comprises exposing the fluid a substituted cyclodextrin;

and wherein harvest and clarification occur before a unit operation comprising capture;
and wherein capture occurs before a unit operation comprising polish chromatography and before a unit operation comprising filtering;

3. recovering a purified biopharmaceutical.

8. The method according to any of claims 1 to 7, wherein said substituted cyclodextrin is substituted with alkyl groups, hydroxyalkyl groups, or sulfoalkyl groups.

9. The method of claim 8, wherein said substituted cyclodextrin is substituted with alkyl groups, hydroxyalkyl groups, or sulfoalkyl groups having 1 to 5 carbon atoms.

10. The method according to any of claims 1 to 9, wherein said substituted cyclodextrin is selected from hydroxypropyl-cyclodextrin, methyl-cyclodextrin, sulfobutyl ether cyclodextrin, or from a mixture thereof.

11. The method of claim 10, wherein said substituted cyclodextrin is selected from hydroxypropyl-beta-cyclodextrin (HPBCD), methyl-beta-cyclodextrin, sulfobutyl ether beta-cyclodextrin, or from a mixture thereof.

12. The method according to any of claims 1 to 11, wherein said substituted cyclodextrin comprises HPBCD.

13. The method according to any of claims 1 to 12, wherein said biopharmaceutical is a therapeutic protein.

14. The method according to any of claims 1 to 13, wherein said method excludes the use of Triton X-100.

[Fig. 1] **Process steps and samples collected and tested in the Viral Clearance Study (VCS) (Example section A-II, 6.2)**

[Fig. 2] **pH inactivation – HPBCD (KLEPTOSE® HP Biopharma) with 15% (w/v) (Example section A-III, 3)**

| Sample No. | No.of wells (mL) | Volume added per well | PFU Well 1 (PFU/0.5 mL) | PFU Well 2 (PFU/0.5 mL) | PFU Well 3 (PFU/0.5 mL) | Mean PFU (PFU/0.5 mL) | 95% Conf. Mean | Dilution (fold) | Virus Titer (PFU/mL) | Virus Titer (Log PFU/mL) | pH Adjust | Dilution adjust | Volume adjust (mL) | Adjusted Titer (PFU) | 95% Conf. of titer | Log 10 Adjusted Titer (PFU) | 95% Conf of Log 10 Adjusted titer | Log 10 Reduction | 95% Conf. of Log 10 Reduction |
|---|---|---|---|---|---|---|---|---|---|---|---|---|---|---|---|---|---|---|---|
| U-22271/009 | 3 | 0.5 | 15 | 12 | 14 | 13.67 | 4.49 | 10000 | 2740000.00 | 6.44 | 1.000 | 50.0 | 100.0 | 1.37E+09 | 3.79E+08 | 9.14 | 0.11 | NA | NA |
| U-22271/010 | 3 | 0.5 | 10 | 8 | 7 | 8.33 | 3.79 | 10000 | 166000.00 | 5.22 | 1.000 | 50.0 | 100.0 | 8.33E+08 | 3.79E+08 | 8.92 | 0.16 | NA | NA |
| U-22271/003 | 3 | 0.5 | 0 | 0 | 0 | 0.00 | NA | 10 | 2.00 | 0.30 | 1.004 | 50.0 | 100.0 | 1.00E+04 | NA | ≥4.00 | NA | ≥5.13 | 0.11 |
| U-22271/007 | 3 | 0.5 | 0 | 0 | 0 | 0.00 | NA | 10 | 2.00 | 0.30 | 1.004 | 50.0 | 100.0 | 1.00E+04 | NA | ≥4.00 | NA | ≥5.13 | 0.11 |
| U-22271/004 | 3 | 0.5 | 0 | 0 | 0 | 0.00 | NA | 10 | 2.00 | 0.30 | 1.004 | 50.0 | 100.0 | 1.00E+04 | NA | ≥4.00 | NA | ≥5.13 | 0.11 |
| U-22271/008 | 3 | 0.1 | 0 | 0 | 0 | 0.00 | NA | 10 | 2.00 | 0.30 | 1.004 | 50.0 | 100.0 | 1.00E+04 | NA | ≥4.00 | NA | ≥5.13 | 0.11 |

U-22271/009  Load control with media
U-22271/010  Processing control with media
U-22271/003  Load Control (15%)
U-22271/004  Processing control with 15%
U-22271/007  T=0 (15%)
U-22271/008  T=60 (15%)

**Note:**

Value 2.00 denotes limit of detection of the assay.

Limit of detection (per sample) = - [ln (0.05)/number of wells plated] x (dilution/volume plated per well)
= - [ln (0.05)/3]*(1/0.5),
= 2.00

[Fig. 3] **pH inactivation – HPBCD (KLEPTOSE® HP Biopharma) with 10% (w/v) (Example section A-III, 3)**

| Sample No. | No. of wells | Volume added per well | PFU Well 1 (PFU/0.5 mL) | PFU Well 2 (PFU/0.5 mL) | PFU Well 3 (PFU/0.5 mL) | Mean PFU (PFU/0.5 mL) | 95% Conf. Mean | Dilution (fold) | Virus Titer (PFU/mL) | Virus Titer (Log PFU/mL) | pH Adjust | Dilution adjust | Volume adjust (mL) | Adjusted Titer (PFU) | 95% Conf. of titer | Log 10 Adjusted Titer (PFU) | 95% Conf. of Log 10 Adjusted titer | Log 10 Reduction | 95% Conf. of Log 10 Reduction pH |
|---|---|---|---|---|---|---|---|---|---|---|---|---|---|---|---|---|---|---|---|
| U-22271/009 | 3 | 0.5 | 15 | 12 | 14 | 13.67 | 3.79 | 10000 | 273400.00 | 5.44 | 1.000 | 50.0 | 100.0 | 1.37E+09 | 3.79E+08 | 9.14 | 0.11 | NA | NA |
| U-22271/010 | 3 | 0.5 | 10 | 8 | 7 | 8.33 | 3.79 | 10000 | 166600.00 | 5.22 | 1.000 | 50.0 | 100.0 | 8.33E+08 | 3.79E+08 | 8.92 | 0.16 | NA | NA |
| U-22271/001 | 3 | 0.5 | 0 | 0 | 0 | 0.00 | NA | 10 | 2.00 | 0.30 | 1.004 | 50.0 | 100.0 | 1.00E+04 | NA | ≥4.00 | NA | ≥5.13 | 0.11 |
| U-22271/002 | 3 | 0.5 | 0 | 0 | 0 | 0.00 | NA | 10 | 2.00 | 0.30 | 1.004 | 50.0 | 100.0 | 1.00E+04 | NA | ≥4.00 | NA | ≥5.13 | 0.11 |
| U-22271/005 | 3 | 0.5 | 0 | 0 | 0 | 0.00 | NA | 10 | 2.00 | 0.30 | 1.004 | 50.0 | 100.0 | 1.00E+04 | NA | ≥4.00 | NA | ≥5.13 | 0.11 |
| U-22271/006 | 3 | 0.5 | 0 | 0 | 0 | 0.00 | NA | 10 | 2.00 | 0.30 | 1.004 | 50.0 | 100.0 | 1.00E+04 | NA | ≥4.00 | NA | ≥5.13 | 0.11 |

**Note:**

U-22271/009  Load control with media

U-22271/010  Processing control with media

U-22271/001  Load Control (10%)

U-22271/002  Processing control with 10%

U-22271/005  T=0 (10%)

U-22271/006  T=60 (10%)

* Value 2.00 denotes limit of detection of the assay. Refer Note below Table-11 for further details

[Fig. 4] **Viral inactivation (virus inactivation using HPBCD)**
**(Example section B-II, 6.2)**

[Fig. 5] **Clarified Harvest + HPBCD 5% (w/v) Run-1 (Example section B-II, 6.2)**

| Sample No. | No. of wells | Volume added per well (mL) | PFU Well 1 (PFU/0.5 mL) | PFU Well 2 (PFU/0.5 mL) | PFU Well 3 (PFU/0.5 mL) | Mean PFU (PFU/0.5 mL) | 95% Conf. Mean | Dilution (fold) | Virus Titer (PFU/mL) | Virus Titer (Log PFU/mL) |
|---|---|---|---|---|---|---|---|---|---|---|
| U-23273/001 | 3 | 0.5 | 6 | 6 | 5 | 5.67 | 1.43 | 100000 | 1134000.00 | 6.05 |
| U-23273/002 | 3 | 0.5 | 3 | 2 | 3 | 2.67 | 1.43 | 100000 | 534000.00 | 5.73 |
| U-23273/005 | 3 | 0.5 | 22 | 21 | 24 | 22.33 | 3.79 | 10 | 446.60 | 2.65 |
| U-23273/006 | 3 | 0.5 | 19 | 18 | 20 | 19.00 | 2.48 | 10 | 380.00 | 2.58 |

| Sample No. | pH Adjust | Dilution adjust | Volume adjust (mL) | Adjusted Titer (PFU) | 95% Conf. of titer | Log 10 Adjusted Titer (PFU) | 95% Conf. of Log 10 Adjusted titer | Log 10 Reduction | 95% Conf. of Log 10 Reduction |
|---|---|---|---|---|---|---|---|---|---|
| U-23273/001 | 1.0000 | 1.00 | 10.00 | 1.13E+07 | 2.87E+06 | 7.05 | 0.10 | NA | NA |
| U-23273/002 | 1.0000 | 1.00 | 10.00 | 5.34E+06 | 2.87E+06 | 6.73 | 0.19 | NA | NA |
| U-23273/005 | 1.0000 | 10.00 | 50.00 | 2.23E+05 | NA | 5.35 | NA | 1.71 | 0.10 |
| U-23273/006 | 1.0000 | 10.00 | 50.00 | 1.90E+05 | NA | 5.28 | NA | 1.78 | 0.10 |

U-23273/001= Clarified harvest Load
U-23273/002= Clarified harvest Processing Control,
U-23273/005= Clarified harvest + 5% HPBCD T=15 minutes
U-23273/006 = Clarified harvest + 5% HPBCD T=60 minutes

[Fig. 6] **Clarified Harvest + HPBCD 5% (w/v) Run-2 (Example section B-II, 6.2)**

| Sample No. | No. of wells | Volume added per well (mL) | PFU Well 1 (PFU/0.5 mL) | PFU Well 2 (PFU/0.5 mL) | PFU Well 3 (PFU/0.5 mL) | Mean PFU (PFU/0.5 mL) | 95% Conf. Mean | Dilution (fold) | Virus Titer (PFU/mL) | Virus Titer (Log PFU/mL) |
|---|---|---|---|---|---|---|---|---|---|---|
| U-23273/017 | 3 | 0.5 | 5 | 4 | 4 | 4.33 | 1.43 | 100000 | 866000.00 | 5.94 |
| U-23273/018 | 3 | 0.5 | 4 | 3 | 3 | 3.33 | 1.43 | 100000 | 666000.00 | 5.82 |
| U-23273/021 | 3 | 0.5 | 18 | 25 | 19 | 20.67 | 9.40 | 10 | 413.40 | 2.62 |
| U-23273/022 | 3 | 0.5 | 17 | 14 | 16 | 15.67 | 3.79 | 10 | 313.40 | 2.50 |

| Sample No. | pH Adjust | Dilution adjust | Volume adjust (mL) | Adjusted Titer (PFU) | 95% Conf. of titer | Log 10 Adjusted Titer (PFU) | 95% Conf. of Log 10 Adjusted titer | Log 10 Reduction | 95% Conf. of Log 10 Reduction |
|---|---|---|---|---|---|---|---|---|---|
| U-23273/017 | 1.0000 | 1.00 | 10.00 | 8.66E+06 | 2.87E+06 | 6.94 | 0.12 | NA | NA |
| U-23273/018 | 1.0000 | 1.00 | 10.00 | 6.66E+06 | 2.87E+06 | 6.82 | 0.16 | NA | NA |
| U-23273/021 | 1.0000 | 10.00 | 50.00 | 2.07E+05 | NA | 5.32 | NA | 1.62 | 0.12 |
| U-23273/022 | 1.0000 | 10.00 | 50.00 | 1.57E+05 | NA | 5.20 | NA | 1.74 | 0.12 |

U-23273/017   Clarified harvest Load
U-23273/018   Clarified harvest Processing Control
U-23273/021   Clarified harvest + 5% HPBCD T=15 minutes
U-23273/022   Clarified harvest + 5% HPBCD T=60 minutes

| Sample No. | No.of wells | Volume added per well (mL) | PFU Well 1 (PFU/0.5 mL) | PFU Well 2 (PFU/0.5 mL) | PFU Well 3 (PFU/0.5 mL) | Mean PFU (PFU/0.5 mL) | 95% Conf. Mean | Dilution (fold) | Virus Titer (PFU/mL) | Virus Titer (Log PFU/mL) |
|---|---|---|---|---|---|---|---|---|---|---|
| U-23273/001 | 3 | 0.5 | 6 | 6 | 5 | 5.67 | 1.43 | 100000 | 1134000.00 | 6.05 |
| U-23273/002 | 3 | 0.5 | 3 | 2 | 3 | 2.67 | 1.43 | 100000 | 534000.00 | 5.73 |
| U-23273/009 | 3 | 0.5 | 8 | 6 | 6 | 6.67 | NA | 1 | 13.34 | 1.13 |
| U-23273/010* | 60 | 0.5 | 0 X 60 wells | | | 0.00 | NA | 1 | 0.10 | -1.00 |

| Sample No. | pH Adjust | Dilution adjust | Volume adjust (mL) | Adjusted Titer (PFU) | 95% Conf. of titer | Log 10 Adjusted Titer (PFU) | 95% Conf. of Log 10 Adjusted titer | Log 10 Reduction | 95% Conf. of Log 10 Reduction |
|---|---|---|---|---|---|---|---|---|---|
| U-23273/001 | 1.0000 | 1.00 | 10.00 | 1.13E+07 | 2.87E+06 | 7.05 | 0.10 | NA | NA |
| U-23273/002 | 1.0000 | 1.00 | 10.00 | 5.34E+06 | 2.87E+06 | 6.73 | 0.19 | NA | NA |
| U-23273/009 | 1.0000 | 10.00 | 50.00 | 6.67E+03 | NA | 3.82 | NA | 3.23 | 0.10 |
| U-23273/010* | 1.0000 | 10.00 | 50.00 | 4.99E+01 | NA | 1.70 | NA | 5.36 | 0.10 |

U-23273/001   Clarified harvest Load
U-23273/002   Clarified harvest Processing Control
U-23273/009   Clarified harvest + 10% HPBCD T=15 minutes
U-23273/010*  Clarified harvest + 10% HPBCD T=60 minutes (Large Volume Plating)

## [Fig. 8] Clarified Harvest + HPBCD 10% (w/v) Run-2 (Example section B-II, 6.2)

| Sample No. | No. of wells | Volume added per well (mL) | PFU Well 1 (PFU/0.5 mL) | PFU Well 2 (PFU/0.5 mL) | PFU Well 3 (PFU/0.5 mL) | Mean PFU (PFU/0.5 mL) | 95% Conf. Mean | Dilution (fold) | Virus Titer (PFU/mL) | Virus Titer (Log PFU/mL) |
|---|---|---|---|---|---|---|---|---|---|---|
| U-23273/017 | 3 | 0.5 | 5 | 4 | 4 | 4.33 | 1.43 | 100000 | 866000.00 | 5.94 |
| U-23273/018 | 3 | 0.5 | 4 | 3 | 3 | 3.33 | 1.43 | 100000 | 666000.00 | 5.82 |
| U-23273/025 | 3 | 0.5 | 12 | 9 | 10 | 0.5 | 3.79 | 1 | 20.66 | 1.32 |
| U-23273/026 | 60 | 0.5 | 0 X 60 wells | | | 0.00 | NA | 1 | 0.10 | -1.00 |

| Sample No. | pH Adjust | Dilution adjust | Volume adjust (mL) | Adjusted Titer (PFU) | 95% Conf. of titer | Log 10 Adjusted Titer (PFU) | 95% Conf. of Log 10 Adjusted titer | Log 10 Reduction | 95% Conf. of Log 10 Reduction |
|---|---|---|---|---|---|---|---|---|---|
| U-23273/017 | 1.0000 | 1.00 | 10.00 | 8.66E+06 | 2.87E+06 | 6.94 | 0.12 | NA | NA |
| U-23273/018 | 1.0000 | 1.00 | 10.00 | 6.66E+06 | 2.87E+06 | 6.82 | 0.16 | NA | NA |
| U-23273/025 | 1.0000 | 10.00 | 50.00 | 1.03E+04 | NA | 4.01 | NA | 2.92 | 0.12 |
| U-23273/026 | 1.0000 | 10.00 | 50.00 | 4.99E+01 | NA | 1.70 | NA | 5.24 | 0.12 |

U-23273/017   Clarified harvest Load
U-23273/018   Clarified harvest Processing Control
U-23273/025   Clarified harvest + 10% HPBCD T=15 minutes
U-23273/026*   Clarified harvest + 10% HPBCD T=60 minutes (Large Volume Plating)

## [Fig. 9] Pro A Neutralized Eluate + HPBCD 5% (w/v) Run-1 (Example section B-II, 6.2)

| Sample No. | No. of wells | Volume added per well (mL) | PFU Well 1 (PFU/0.5 mL) | PFU Well 2 (PFU/0.5 mL) | PFU Well 3 (PFU/0.5 mL) | Mean PFU (PFU/0.5 mL) | 95% Conf. Mean | Dilution (fold) | Virus Titer (PFU/mL) | Virus Titer (Log PFU/mL) |
|---|---|---|---|---|---|---|---|---|---|---|
| U-23273/003 | 3 | 0.5 | 8 | 7 | 6 | 7.00 | 2.48 | 100000 | 1400000.00 | 6.15 |
| U-23273/004 | 3 | 0.5 | 3 | 2 | 3 | 2.67 | 1.43 | 100000 | 534000.00 | 5.73 |
| U-23273/007 | 3 | 0.5 | 20 | 22 | 24 | 22.00 | 4.97 | 10 | 440.00 | 2.64 |
| U-23273/008 | 3 | 0.5 | 2 | 2 | 2 | 2.00 | 0.00 | 100 | 400.00 | 2.60 |

| Sample No. | pH Adjust | Dilution adjust | Volume adjust (mL) | Adjusted Titer (PFU) | 95% Conf. of titer | Log 10 Adjusted Titer (PFU) | 95% Conf. of Log 10 Adjusted titer | Log 10 Reduction | 95% Conf. of Log 10 Reduction |
|---|---|---|---|---|---|---|---|---|---|
| U-23273/003 | 1.0000 | 1.00 | 10.00 | 1.40E+07 | 4.97E+07 | 7.15 | 0.13 | NA | NA |
| U-23273/004 | 1.0000 | 1.00 | 10.00 | 5.34E+06 | 2.87E+06 | 6.73 | 0.19 | NA | NA |
| U-23273/007 | 1.0000 | 10.00 | 50.00 | 2.20E+05 | NA | 5.34 | NA | 1.80 | 0.13 |
| U-23273/008 | 1.0000 | 10.00 | 50.00 | 2.00E+05 | NA | 5.30 | NA | 1.85 | 0.13 |

U-23273/003  Pro A Neutralized Elute Load
U-23273/004  Pro A Neutralized Elute Processing Control
U-23273/007  Pro A Neutralized Elute + 5% HPBCD T=15 minutes
U-23273/008  Pro A Neutralized Elute+ 5% HPBCD T=60 minutes

## [Fig. 10] Pro A Neutralized Eluate + HPBCD 5% (w/v) Run-2 (Example section B-II, 6.2)

| Sample No. | No. of wells | Volume added per well (mL) | PFU Well 1 (PFU/0.5 mL) | PFU Well 2 (PFU/0.5 mL) | PFU Well 3 (PFU/0.5 mL) | Mean PFU (PFU/0.5 mL) | 95% Conf. Mean | Dilution (fold) | Virus Titer (PFU/mL) | Virus Titer (Log PFU/mL) |
|---|---|---|---|---|---|---|---|---|---|---|
| U-23273/019 | 3 | 0.5 | 5 | 6 | 5 | 5.33 | 1.43 | 100000 | 1066000.00 | 6.03 |
| U-23273/020 | 3 | 0.5 | 25 | 27 | 32 | 28.00 | 8.95 | 10000 | 560000.00 | 5.75 |
| U-23273/023 | 3 | 0.5 | 25 | 27 | 18 | 23.33 | 11.73 | 10 | 466.60 | 2.67 |
| U-23273/024 | 3 | 0.5 | 10 | 8 | 12 | 10.00 | 4.97 | 10 | 200.00 | 2.30 |

| Sample No. | pH Adjust | Dilution adjust | Volume adjust (mL) | Adjusted Titer (PFU) | 95% Conf. of titer | Log 10 Adjusted Titer (PFU) | 95% Conf. of Log 10 Adjusted titer | Log 10 Reduction | 95% Conf. of Log 10 Reduction |
|---|---|---|---|---|---|---|---|---|---|
| U-23273/019 | 1.0000 | 1.00 | 10.00 | 1.07E+07 | 2.87E+06 | 7.03 | 0.10 | NA | NA |
| U-23273/020 | 1.0000 | 1.00 | 10.00 | 5.60E+06 | 1.79E+06 | 6.75 | 0.12 | NA | NA |
| U-23273/023 | 1.0000 | 10.00 | 50.00 | 2.33E+05 | NA | 5.37 | NA | 1.66 | 0.10 |
| U-23273/024 | 1.0000 | 10.00 | 50.00 | 1.00E+05 | NA | 5.00 | NA | 2.03 | 0.10 |

U-23273/019    Pro A Neutralized Elute Load
U-23273/020    Pro A Neutralized Elute Processing Control
U-23273/023    Pro A Neutralized Elute + 5% HPBCD T=15 minutes
U-23273/024    Pro A Neutralized Elute+ 5% HPBCD T=60 minutes

**[Fig. 11] Pro A Neutralized Eluate + HPBCD 10% (w/v) Run-1 (Example section B-II, 6.2)**

| Sample No. | No. of wells | Volume added per well (mL) | PFU Well 1 (PFU/0.5 mL) | PFU Well 2 (PFU/0.5 mL) | PFU Well 3 (PFU/0.5 mL) | Mean PFU (PFU/0.5 mL) | 95% Conf. Mean | Dilution (fold) | Virus Titer (PFU/mL) | Virus Titer (Log PFU/mL) |
|---|---|---|---|---|---|---|---|---|---|---|
| U-23273/003 | 3 | 0.5 | 8 | 7 | 6 | 7.00 | 2.48 | 1000000 | 1400000.00 | 6.15 |
| U-23273/004 | 3 | 0.5 | 3 | 2 | 3 | 2.67 | 1.43 | 100000 | 534000.00 | 5.73 |
| U-23273/011 | 3 | 0.5 | 1 | 1 | 1 | 1.00 | 0.00 | 10 | 20.00 | 1.30 |
| U-23273/012* | 60 | 0.5 | 0 X 60 wells | | | 0.00 | NA | 1 | 0.10 | -1.00 |

| Sample No. | pH Adjust | Dilution adjust | Volume adjust (mL) | Adjusted Titer (PFU) | 95% Conf. of titer | Log 10 Adjusted Titer (PFU) | 95% Conf. of Log 10 Adjusted titer | Log 10 Reduction | 95% Conf. of Log 10 Reduction |
|---|---|---|---|---|---|---|---|---|---|
| U-23273/003 | 1.0000 | 1.00 | 10.00 | 1.40E+07 | 4.97E+07 | 7.15 | 0.13 | NA | NA |
| U-23273/004 | 1.0000 | 1.00 | 10.00 | 5.34E+06 | 2.87E+06 | 6.73 | 0.19 | NA | NA |
| U-23273/011 | 1.0000 | 10.00 | 50.00 | 1.00E+04 | NA | 4.00 | NA | 3.15 | 0.13 |
| U-23273/012* | 1.0000 | 10.00 | 50.00 | 4.99E+01 | NA | 1.70 | NA | 5.45 | 0.13 |

U-23273/003    Pro A Neutralized Elute Load
U-23273/004    Pro A Neutralized Elute Processing Control
U-23273/011    Pro A Neutralized Elute + 10% HPBCD T=15 minutes
U-23273/012*    Pro A Neutralized Elute+ 10% HPBCD T=60 minutes (Large Volume Plating)

[Fig. 12] **Pro A Neutralized Eluate + HPBCD 10% (w/v) Run-2 (Example section B-II, 6.2)**

| Sample No. | No. of wells | Volume added per well (mL) | PFU Well 1 (PFU/0.5 mL) | PFU Well 2 (PFU/0.5 mL) | PFU Well 3 (PFU/0.5 mL) | Mean PFU (PFU/0.5 mL) | 95% Conf. Mean | Dilution (fold) | Virus Titer (PFU/mL) | Virus Titer (Log PFU/mL) |
|---|---|---|---|---|---|---|---|---|---|---|
| U-23273/019 | 3 | 0.5 | 5 | 6 | 5 | 5.33 | 1.43 | 100000 | 1066000.00 | 6.03 |
| U-23273/020 | 3 | 0.5 | 25 | 27 | 32 | 28.00 | 8.95 | 10000 | 560000.00 | 5.75 |
| U-23273/027 | 3 | 0.5 | 14 | 15 | 19 | 16.00 | 6.57 | 1 | 32.00 | 1.51 |
| U-23273/028* | 60 | 0.5 | 0 X 60 wells | | | 0.00 | NA | 1 | 0.10 | -1.00 |

| Sample No. | pH Adjust | Dilution adjust | Volume adjust (mL) | Adjusted Titer (PFU) | 95% Conf. of titer | Log 10 Adjusted Titer (PFU) | 95% Conf. of Log 10 Adjusted titer | Log 10 Reduction | 95% Conf. of Log 10 Reduction |
|---|---|---|---|---|---|---|---|---|---|
| U-23273/019 | 1.0000 | 1.00 | 10.00 | 1.07E+07 | 2.87E+06 | 7.03 | 0.10 | NA | NA |
| U-23273/020 | 1.0000 | 1.00 | 10.00 | 5.60E+06 | 1.79E+06 | 6.75 | 0.12 | NA | NA |
| U-23273/027 | 1.0000 | 10.00 | 50.00 | 1.60E+04 | NA | 4.20 | NA | 2.82 | 0.10 |
| U-23273/028* | 1.0000 | 10.00 | 50.00 | 4.99E+01 | NA | 1.70 | NA | 5.33 | 0.10 |

U-23273/019  Pro A Neutralized Elute Load
U-23273/020  Pro A Neutralized Elute Processing Control
U-23273/027  Pro A Neutralized Elute + 10% HPBCD T=15 minutes
U-23273/028*  Pro A Neutralized Elute+ 10% HPBCD T=60 minutes (Large Volume Plating)

[Fig. 13] **Clarified Harvest + 0.3 % (w/v) TNBP + 1% (w/v) Triton X-100 Run-1 (Example section B-II, 6.2)**

| Sample No. | No. of wells | Volume added per well (mL) | PFU Well 1 (PFU/0.5 mL) | PFU Well 2 (PFU/0.5 mL) | PFU Well 3 (PFU/0.5 mL) | Mean PFU (PFU/0.5 mL) | 95% Conf. Mean | Dilution (fold) | Virus Titer (PFU/mL) | Virus Titer (Log PFU/mL) |
|---|---|---|---|---|---|---|---|---|---|---|
| U-23273/001 | 3 | 0.5 | 6 | 6 | 5 | 5.67 | 1.43 | 100000 | 1134000.00 | 6.05 |
| U-23273/002 | 3 | 0.5 | 3 | 2 | 3 | 2.67 | 1.43 | 100000 | 534000.00 | 5.73 |
| U-23273/013* | 60 | 0.5 | 0 X 60 wells | | | 0.00 | NA | 1 | 0.10 | -1.00 |
| U-23273/014* | 60 | 0.5 | 0 X 60 wells | | | 0.00 | NA | 1 | 0.10 | -1.00 |

| Sample No. | pH Adjust | Dilution adjust | Volume adjust (mL) | Adjusted Titer (PFU) | 95% Conf. of titer | Log 10 Adjusted Titer (PFU) | 95% Conf. of Log 10 Adjusted titer | Log 10 Reduction | 95% Conf. of Log 10 Reduction |
|---|---|---|---|---|---|---|---|---|---|
| U-23273/001 | 1.0000 | 1.00 | 10.00 | 1.13E+07 | 2.87E+06 | 7.05 | 0.10 | NA | NA |
| U-23273/002 | 1.0000 | 1.00 | 10.00 | 5.34E+06 | 2.87E+06 | 6.73 | 0.19 | NA | NA |
| U-23273/013* | 1.0000 | 200.00 | 50.00 | 9.99E+02 | NA | 3.00 | NA | 4.06 | 0.10 |
| U-23273/014* | 1.0000 | 200.00 | 50.00 | 9.99E+02 | NA | 3.00 | NA | 4.06 | 0.19 |

U-23273/001 Clarified Harvest Load
U-23273/002 Clarified Harvest Processing Control
U-23273/013* Clarified harvest+ 0.3%TNBP + 1%Triton-X T=15 minutes (Large Volume Plating)
U-23273/014* Clarified harvest + 0.3%TNBP + 1%Triton-X T=60 minutes (Large Volume Plating)

[Fig. 14] **PA Neutralized Eluate + 0.3 % (w/v) TNBP + 1% (w/v) Triton X-100 Run-1 (Example section B-II, 6.2)**

| Sample No. | No. of wells | Volume added per well (mL) | PFU Well 1 (PFU/0.5 mL) | PFU Well 2 (PFU/0.5 mL) | PFU Well 3 (PFU/0.5 mL) | Mean PFU (PFU/0.5 mL) | 95% Conf. Mean | Dilution (fold) | Virus Titer (PFU/mL) | Virus Titer (Log PFU/mL) |
|---|---|---|---|---|---|---|---|---|---|---|
| U-23273/003 | 3 | 0.5 | 8 | 7 | 6 | 7.00 | 2.48 | 100000 | 1400000.00 | 6.15 |
| U-23273/004 | 3 | 0.5 | 3 | 2 | 3 | 2.67 | 1.43 | 100000 | 534000.00 | 5.73 |
| U-23273/015* | 60 | 0.5 | 0 X 60 wells | | | 0.00 | NA | 1 | 0.10 | -1.00 |
| U-23273/016* | 60 | 0.5 | 0 X 60 wells | | | 0.00 | NA | 1 | 0.10 | -1.00 |

| Sample No. | Volume adjust (mL) | Adjusted Titer (PFU) | 95% Conf. of titer | Log 10 Adjusted Titer (PFU) | 95% Conf. of Log 10 Adjusted titer | Log 10 Reduction | 95% Conf. of Log 10 Reduction |
|---|---|---|---|---|---|---|---|
| U-23273/003 | 10.00 | 1.40E+07 | 4.97E+06 | 7.15 | 0.13 | NA | NA |
| U-23273/004 | 10.00 | 5.34E+06 | 2.87E+06 | 6.73 | 0.19 | NA | NA |
| U-23273/015* | 50.00 | 9.99E+02 | NA | 3.00 | NA | 4.15 | 0.13 |
| U-23273/016* | 50.00 | 9.99E+02 | NA | 3.00 | NA | 4.15 | 0.19 |

U-23273/003    Pro A Neutralized Elute Load
U-23273/004    Pro A Neutralized Elute Processing Control
U-23273/015*   Pro A Neutralized Elute+ 0.3%TNBP + 1%Triton-X T=15 minutes (Large Volume Plating)
U-23273/016*   Pro A Neutralized Elute + 0.3%TNBP + 1%Triton-X T=60 minutes (Large Volume Plating)

[Fig. 15] **Virus inactivation on Clarified Harvest fluid (Example section B-II, 6.2)**

[Fig. 16] **Virus inactivation on Protein A eluate (Example section B-II, 6.2)**

[Fig. 17] **Quantification of host cell protein (HCP) after each chromatography step for the initial HCCF load with varying concentrations of HPBCD (Example section C-III)**

HCP Conc.

KLEPTOSE® HP Conc.

Protein A Input
Protein A Eluate
AEX FT
CEX Eluate

[Fig. 18] **Quantification of host cell DNA (HCDNA) after each chromatography step for the initial HCCF load with varying concentrations of HPBCD (Example section C-III)**

HCDNA Conc.

KLEPTOSE® HP Conc.

Protein A Input
Protein A Eluate
AEX FT
CEX Eluate

[Fig. 19] **Quantification of residual protein A after each chromatography step for the initial HCCF load with varying concentrations of HPBCD (Example section C-III)**

Residual Protein A Conc.

□ Protein A Input
▨ Protein A Eluate
■ AEX FT
▨ CEX Eluate

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 408 280 B1 (ELANCO US INC [US]) 25 November 2020 (2020-11-25) * [0001],[0010], claims 1-14 * | 1-11,13, 14 | INV. C07K1/14 |
| X | BEZERRA BRUNO BRAZ ET AL: "Hydroxypropyl-beta-cyclodextrin (HP-BCD) inhibits SARS-CoV-2 replication and virus-induced inflammatory cytokines", ANTIVIRAL RESEARCH, ELSEVIER BV, NL, vol. 205, 4 July 2022 (2022-07-04), XP087148679, ISSN: 0166-3542, DOI: 10.1016/J.ANTIVIRAL.2022.105373 [retrieved on 2022-07-04] * abstract; page 4, col. 2, par. 2 and 3 * | 1-14 | |
| A | WO 2007/135523 A2 (VIROBLOCK S A [CH]; PELET THIERRY [CH]; WALLACH DONALD F H [CH]) 29 November 2007 (2007-11-29) * page 11 line 7-page 12 line 11; claims 1-19 * | 1-14 | |
| A | US 2022/106573 A1 (PERELL GABRIELLA [US] ET AL) 7 April 2022 (2022-04-07) * [0002]-[0005] * | 1-14 | |

TECHNICAL FIELDS SEARCHED (IPC)

C12N
C07K

The present search report has been drawn up for all claims

| Place of search | Date of completion of the search | Examiner |
|---|---|---|
| Munich | 4 December 2024 | Dumont, Elisabeth |

## ANNEX TO THE EUROPEAN SEARCH REPORT
## ON EUROPEAN PATENT APPLICATION NO.

EP 24 30 6164

This annex lists the patent family members relating to the patent documents cited in the above-mentioned European search report.
The members are as contained in the European Patent Office EDP file on
The European Patent Office is in no way liable for these particulars which are merely given for the purpose of information.

04-12-2024

| Patent document cited in search report | | Publication date | Patent family member(s) | | Publication date |
|---|---|---|---|---|---|
| EP 3408280 | B1 | 25-11-2020 | AR | 107262 A1 | 11-04-2018 |
| | | | AU | 2017211040 A1 | 28-06-2018 |
| | | | BR | 112018014542 A2 | 11-12-2018 |
| | | | CA | 3008965 A1 | 03-08-2017 |
| | | | CL | 2018001978 A1 | 26-10-2018 |
| | | | CN | 108602860 A | 28-09-2018 |
| | | | DK | 3408280 T3 | 04-01-2021 |
| | | | EA | 201891371 A1 | 31-01-2019 |
| | | | EP | 3408280 A1 | 05-12-2018 |
| | | | ES | 2839623 T3 | 05-07-2021 |
| | | | JP | 6648286 B2 | 14-02-2020 |
| | | | JP | 2019506860 A | 14-03-2019 |
| | | | KR | 20180096775 A | 29-08-2018 |
| | | | NZ | 743601 A | 27-03-2020 |
| | | | PL | 3408280 T3 | 31-05-2021 |
| | | | TW | 201733615 A | 01-10-2017 |
| | | | US | 2018360945 A1 | 20-12-2018 |
| | | | WO | 2017132059 A1 | 03-08-2017 |
| | | | ZA | 201804025 B | 26-08-2020 |
| WO 2007135523 | A2 | 29-11-2007 | AU | 2007252967 A1 | 29-11-2007 |
| | | | BR | PI0712074 A2 | 17-01-2012 |
| | | | CA | 2652362 A1 | 29-11-2007 |
| | | | CN | 101448486 A | 03-06-2009 |
| | | | DK | 2023896 T3 | 03-10-2016 |
| | | | EA | 200802358 A1 | 28-04-2009 |
| | | | EP | 2023896 A2 | 18-02-2009 |
| | | | ES | 2590812 T3 | 23-11-2016 |
| | | | HK | 1131554 A1 | 29-01-2010 |
| | | | IL | 195317 A | 27-02-2014 |
| | | | JP | 5259578 B2 | 07-08-2013 |
| | | | JP | 2009537508 A | 29-10-2009 |
| | | | KR | 20090014178 A | 06-02-2009 |
| | | | US | 2010137437 A1 | 03-06-2010 |
| | | | US | 2015065458 A1 | 05-03-2015 |
| | | | WO | 2007135523 A2 | 29-11-2007 |
| US 2022106573 | A1 | 07-04-2022 | AR | 118457 A1 | 06-10-2021 |
| | | | AU | 2020240034 A1 | 19-08-2021 |
| | | | CA | 3133794 A1 | 24-09-2020 |
| | | | CN | 113412270 A | 17-09-2021 |
| | | | CN | 118852319 A | 29-10-2024 |
| | | | EP | 3941928 A1 | 26-01-2022 |
| | | | IL | 285222 A | 30-09-2021 |
| | | | JP | 2022525361 A | 12-05-2022 |
| | | | KR | 20210142670 A | 25-11-2021 |

For more details about this annex : see Official Journal of the European Patent Office, No. 12/82

page 1 of 2

## ANNEX TO THE EUROPEAN SEARCH REPORT
## ON EUROPEAN PATENT APPLICATION NO.

EP 24 30 6164

This annex lists the patent family members relating to the patent documents cited in the above-mentioned European search report.
The members are as contained in the European Patent Office EDP file on
The European Patent Office is in no way liable for these particulars which are merely given for the purpose of information.

04-12-2024

| Patent document cited in search report | Publication date | Patent family member(s) | | Publication date |
|---|---|---|---|---|
| | | MA | 55371 A | 26-01-2022 |
| | | SG | 11202109801Y A | 28-10-2021 |
| | | TW | 202102518 A | 16-01-2021 |
| | | US | 2022106573 A1 | 07-04-2022 |
| | | WO | 2020190985 A1 | 24-09-2020 |

EPO FORM P0459

For more details about this annex : see Official Journal of the European Patent Office, No. 12/82

page 2 of 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Hydroxypropyl Betadex ; Molar substitutio. *USP 41 NF 36 method*, 01 January 2024
- *US monograph*, 01 January 2024
- *European monograph*, 01 January 2024
- Current Protocols in Molecular Biology. Wiley & Sons, 1990

- **SAMBROOK et al.** Molecular Cloning: A Laboratory Manual. Cold Spring Laboratory Press, 1989
- **KAUFMAN, R. L**. *Large Scale Mammalian Cell Culture*, 1990, 15-69